(19) Europäisches Patentamt
European Patent Office
Office européen des brevets

(11) Publication number: 0 166 697
B1

(12) **EUROPEAN PATENT SPECIFICATION**

(45) Date of publication of patent specification: 09.05.90

(21) Application number: 85810293.2

(22) Date of filing: 24.06.85

(51) Int. Cl.$^5$: C 07 D 279/12, C 07 D 417/06, C 07 C 323/22, C 07 C 323/23, A 61 K 31/54 // C07D209/48

# (54) Aroyl substituted dihydro-1,4-thiazines.

(30) Priority: 29.06.84 US 625946

(43) Date of publication of application: 02.01.86 Bulletin 86/01

(45) Publication of the grant of the patent: 09.05.90 Bulletin 90/19

(84) Designated Contracting States: AT BE CH DE FR GB IT LI LU NL SE

(56) References cited:
GB-A-2 089 796
US-A-3 452 001

TETRAHEDRON LETTERS, no. 35, 1967, pages 3439-3442; Pergamon Press Ltd., GB TERUAKI MUKAIYAMA et al.:"The reactions of omega-(phenylthio)acetophenone."

(73) Proprietor: CIBA-GEIGY AG
Klybeckstrasse 141
CH-4002 Basel (CH)

(72) Inventor: Renfroe, Harris Burt
12 Stonehedge Drive
West Nyack New York 10994 (US)

EP 0 166 697 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to compounds of the formula I:

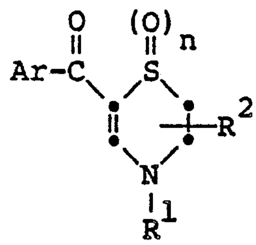

I

wherein Ar is pyridyl, phenyl or phenyl substituted by halogen, halo-$C_{1-4}$-alkyl, $C_{1-4}$-alkoxy or carboxy-$C_{1-4}$-alkyl, n is zero, one or two, $R^1$ is hydrogen, $C_{1-4}$-alkyl, heterocyclyl-$C_{2-4}$-alkyl, amino-$C_{2-4}$-alkyl, $C_{1-4}$-alkylamino-$C_{2-4}$-alkyl, di-$C_{1-4}$-alkylamino-$C_{2-4}$-alkyl or $C_{1-4}$-alkanoyl and $R^2$ is hydrogen, carboxy-$C_{1-4}$-alkyl or $C_{1-4}$-alkoxycarbonyl-$C_{1-4}$-alkyl, with the exception of 5,6-dihydro-1,4(4H)-thiazin-2-yl phenyl ketone and the corresponding sulfoxide and sulfone, and salts of these compounds that have a salt forming group, to pharmaceutical compositions that contain these compounds, to the use of these compounds as a medicament; and to the manufacture of pharmaceutical compositions, and to processes for the manufacture of these compounds.

The invention also relates to a pharmaceutical composition that contains a compound of the formula I, wherein Ar is phenyl, n is zero, one or two, and $R^1$ and $R^2$ are hydrogen, and pharmaceutically acceptable salts of these compounds, to the use of these compounds as a medicament, and to the manufacture of a pharmaceutical composition.

In the specification of the present invention, the term "lower", which is used in connection with groups or radicals, for example lower alkyl, lower alkylene, lower alkoxy, lower alkanoyl etc., means that, unless expressly defined otherwise the groups or radicals so designated contain up to and including 7, and preferably up to and including 4 carbon atoms.

In the compounds of the formula I, n represents especially zero. If n represents 1 and $R^2$ is hydrogen, the compounds of formula I exist as enantiomers. If n represents 1 and $R^2$ is other than hydrogen, the compounds of the formula I also exist as cis/trans-isomers. Stereoisomers as well as cis/trans-isomers of compounds of the formula I also fall within the scope of the present invention.

The generic terms used in the specification of the invention preferably are defined as follows:

Pyridyl is 2-, 3- or 4-pyridyl.

Halogen is for example bromo or iodo or, preferably, chloro or fluoro.

Halo-$C_{1-4}$-alkyl, for example, fluoromethyl, 2,2,2-trichloroethyl, or, preferably, trifluoromethyl.

$C_{1-4}$-Alkoxy is, for example, ethoxy or, preferably methoxy.

Carboxy-$C_{1-4}$-alkyl is, for example, 2-carboxyethyl or, preferably, carboxymethyl.

Phenyl substituted by halogen, halo-$C_{1-4}$-alkyl, $C_{1-4}$-alkoxy or carboxy-$C_{1-4}$-alkyl is, for example, 2-fluoro, 2-chloro, 2-, 3- or 4-trifluoromethyl-, 2- or 3-methoxy- or 2-, 3- or 4-carboxymethylphenyl, or, preferably, 3- or 4-fluoro-, 3- or 4-chloro-, 3,4-dichloro- or 4-methoxyphenyl.

$C_{1-4}$-Alkyl $R^1$ is for example ethyl, n-propyl, isopropyl, n-butyl, tert-butyl, sec-butyl or, preferably, methyl.

Heterocyclyl-$C_{2-4}$-alkyl $R^1$ is, for example, (2-piperazin-1-yl)-ethyl, 2-(1-methylpiperazin-4-yl)-ethyl, 2-(1-(2-hydroxyethyl)-piperazin-4-yl)-ethyl, 2-(morpholin-4-yl)-ethyl, or preferably, 2-(piperid-1-yl)-ethyl.

Amino-$C_{2-4}$-alkyl $R^1$ is, for example, 3-amino-n-propyl or, preferably, 2-aminoethyl.

$C_{1-4}$-Alkylamino-$C_{2-4}$-alkyl $R^1$ is, for example, 3-methylamino-n-propyl or, preferably, 2-methylaminoethyl.

Di-$C_{1-4}$-alkylamino-$C_{2-4}$-alkyl $R^1$ is, for example, 3-dimethylamino-n-propyl or, preferably, 2-dimethylaminoethyl.

$C_{1-4}$-Alkanoyl $R^1$ is, for example, formyl or propionyl or, preferably, acetyl.

Carboxy-$C_{1-4}$-alkyl $R^2$ is, for example, 2-carboxyethyl or, preferably carboxymethyl.

$C_{1-4}$-Alkoxycarbonyl-$C_{1-4}$-alkyl $R^2$ is, for example, 2-methyoxy- or 2-ethoxycarbonylmethyl or, preferably, 2-methoxy- or 2-ethoxycarbonylethyl.

Salts of compounds of the formula I which contain a salt forming group are, in particular, pharmaceutically acceptable non-toxic salts.

Such salts are formed by the carboxy group, when Ar is phenyl substituted by carboxy-$C_{1-4}$-alkyl or $R^2$ is carboxy-$C_{1-4}$-alkyl by addition of a base and are, for example, metal or ammonium salts, for example alkali metal or alkaline earth metal salts, for example sodium, potassium, magnesium or calcium salts, or ammonium salts, which are formed by addition of ammonia or a suitable organic amine, for example, 2-triethylamine, chlorine, morpholine, piperazine, piperidine, dicyclohexylamine, pyridine, collidine, or quinoline.

Salts also formed when $R^1$ represents hydrogen, amino-$C_{2-4}$-alkyl, $C_{1-4}$-alkylamino-$C_{2-4}$-alkyl, di-$C_{1-4}$-alkylamino-$C_{2-4}$-alkyl or heterocyclyl-$C_{2-4}$-alkyl, by addition of an inorganic acid, for example a hydrohalic acid, e.g. hydrochloric acid, sulfuric acid, or phosphoric acid, and are, for example, hydrochlorides, hydrogensulfates, hydrogenphosphates, or dihydrogenphosphates.

The functional groups present in compounds of the formula I, for example the carboxy group, when Ar is phenyl substituted by carboxy-$C_{1-4}$-alkyl or $R^2$ is carboxy-$C_{1-4}$-alkyl, or the secondary amino group, when $R^1$ is hydrogen, are optionally protected by protecting groups which are customarily used in peptide chemistry. These protecting groups protect the functional groups from undesired side reactions, such as acylation, etherification, esterification, oxidation, solovolysis etc. and are removed easily under mild reaction conditions, for example by solvolysis, reduction, photolysis, or enzymatic cleavage.

The protection of functional groups by such protecting groups, the protecting groups themselves, and reactions for their removal, are described, for example, in "Protective Groups in Organic Chemistry", Plenum Press, London and New York 1973, in Greene, Th. W., "Protective Groups in Organic Synthesis", Wiley, New York 1981 in "The Peptides", Vol. I, Schroeder and Luebke, Academic Press, London and New York 1965, and in "Methoden der Organischen Chemie", Houben-Weyl, 4th Edition, Vol. 15/I, Georg Thieme Verlag, Stuttgart 1974.

A protected carboxy group is preferably tert-lower alkoxycarbonyl, for example tert-butoxycarbonyl, benzyloxycarbonyl, 4-nitrobenzyloxycarbonyl or diphenylmethoxycarbonyl.

When a carboxy group is present, this group can also be protected by a group that can be cleaved under physiological conditions, for example enzymatically. Such groups are, for example, lower alkanoyloxy-lower alkoxycarbonyl, for example lower alkanoyloxymethoxycarbonyl or lower alkanoyloxy-ethoxycarbonyl, for example acetoxymethoxycarbonyl, pivaloyloxymethoxycarbonyl or 1-propionyloxy-ethoxycarbonyl, lower alkoxycarbonyloxy-lower alkoxycarbonyl, for example 1-ethoxycarbonyloxy-ethoxycarbonyl or tert-butoxycarbonyloxymethoxycarbonyl, or amino-lower alkanoyloxymethoxycarbonyl, for example glycyloxymethoxycarbony, L-valyloxymethoxycarbonyl, or L-leucyloxymethoxy-carbonyl.

A protected amino group is preferably tert-butoxycarbonylamino (BOC), 4-nitrobenzyloxycarbonyl-amino, diphenylmethoxycarbonylamino, or 2,2,2-trichloroethoxycarbonylamino.

In an alphabetically increasing order of preference, the invention relates to the following compounds of formula I:

a) Compounds of formula I, wherein Ar is pyridyl or phenyl substituted by halogen, for example fluoro or chloro, trifluoromethyl, $C_{1-4}$-alkoxy, for example methoxy, or carboxy-$C_{1-4}$-alkyl, for example, carboxymethyl, n is zero, one or two, $R^1$ is hydrogen, $C_{1-4}$-alkyl, for example, methyl, heterocyclyl-$C_{2-4}$-alkyl, for example 2-(piperid-1-yl)-ethyl, di-$C_{1-4}$-alkylamino-$C_{2-4}$-alkyl, for example 2-dimethylaminoethyl, or $C_{1-4}$-alkanoyl, for example acetyl, and $R^2$ is hydrogen or carboxy-$C_{1-4}$-alkyl, for example carboxymethyl, and pharmaceutically acceptable salts of those compounds that have salt forming groups;

b) Compounds of formula I, wherein Ar represents 3-pyridyl, n is zero, $R^1$ represents hydrogen $C_{1-4}$-alkyl, for example methyl, or $C_{1-4}$-alkanoyl, for example acetyl, and $R^2$ is hydrogen or compound of formula I, wherein Ar is phenyl monosubstituted by halogen, for example fluoro or chloro, or phenyl disubstituted, one substituent being trifluoromethyl or halogen, for example fluoro or chloro, and the other one being halogen, for example fluoro or chloro, wherein n is zero, one or two, $R^1$ represents hydrogen, $C_{1-4}$-alkyl, for example methyl, or $C_{1-4}$-alkanoyl, for example acetyl, and $R^2$ is hydrogen, and pharmaceutically acceptable salts of those compounds that have salt forming groups;

c) Compounds of formula I, wherein Ar is phenyl monosubstituted by halogen, for example fluoro or chloro, or phenyl disubstituted, one substituent being trifluoromethyl or halogen, for example fluoro or chloro, and the other one being halogen, for example fluoro or chloro, n is zero and $R^1$ and $R^2$ are hydrogen.

d) A compound of the formula I, wherein Ar is 4-fluorophenyl, n is zero, and $R^1$ and $R^2$ are hydrogen, being 5,6-dihydro-1,4(4H)-thiazin-2-yl 4-fluorophenyl ketone. The compounds of the invention and the compounds of formula I, wherein Ar is phenyl, n is zero, one or two, and $R^1$ and $R^2$ are hydrogen, exhibit valuable pharmacological properties, especially antirheumatic activity. These properties can be demonstrated by in-vitro or in-vivo tests, using for the latter advantageously mammals, such as mice, rats, guinea pigs or dogs, as test objects. The compounds according to the present invention can be administered to the animals either enterally, preferably orally, parenterally, e.g. subcutaneously or intravenously, or topically, for example in the form of oil solutions or starchy suspensions. The applied dosage may range between 0.1 and 100 mg/kg per day, preferably between about 1 and 50 mg/kg per day. The tests for the screening are chosen from the following assay methods:

*1. Developing Adjuvant Arthritis Test:* Charles River (COBS-DC) male rats weighing 325—400 g are injected with 0.05 ml of saline/oil emulsion of *Mycobacterium tuberculosis* (Difco, 6 mg/ml) into the subplantar region of the right hind paw (day 1). On day 3 after injection, animals showing a good inflammatory response are selected and assigned to treatment groups of nine rats each with three rats per cage. The test compound is suspended in an aqueous cornstarch vehicle [3% (w/v) cornstarch, 5% (w/v) polyethylene glycol 400 and 0.34% (w/v) Tween 80®] by grinding and mixing with a ground glass homogenizer. Vehicle, and test compound are administered orally (10 ml/kg) once daily for 12 days beginning on the third day after adjuvant injection.

Left hind paws are measured by mercury displacement on day 15. Mercury displacement for each

group on day 15 is expressed as mean displacement (M). Significance is determined by comparing treated means (MTR) with adjuvant control mean (MAC). Body weights are reported as differences between day 3 and day 15. The percent protection (%P) is calculated on swelling (Mean displacement of paws corrected for normal control:

$$\%P = \frac{MAC - MTR}{MAC - MNC} \times 100$$

(MAC = Mean Adjuvant Control Day 15, MTR = Mean Treated Day 15, MNC = Mean Norman Control Day 15).

Experiments are run at 12.5, 25, and 50 mg/kg p.o. with 5,6-dihydro-1,4(4H)-thiazin-2-yl, 4-fluorophenyl ketone as representative test compound and replicated. All treatments are effective in suppressing paw volume as compared to the development of this polyarthritis in adjuvant treated controls.

2. *Carrageenan-Induced Pleurisy Test:* Sprague Dawley male rats (Charles River) weighing 300—325 g are used. Pleurisy is induced by injecting 0.3 ml of 1% sterile, aqueous solution of carrageenan into the pleural cavity of the rats. Carrageenan is solubilized in distilled water by autoclaving. Seventy-two hours after the carrageenan injection, the inflammatory exudate cells are collected with a Pasteur pipette by washing the cavity with 4 ml of medium 199 containing 5 units heparin per ml. Heparin is used to prevent cell aggregation. Any animals with blood in the pleural cavity are rejected. Total leukocyte counts are performed with a Coulter counter, Model $ZB_1$, using a 20 microliter aliquot of pleural exudate in 10 ml of Isotone®. Differential counts of exudate smears are determined by standard procedures.

The rats are distributed into treatment groups of 10—12 with two or three rats per cage. Each rat receives a marking on the tail for proper identification. The test compound is suspended in an aqueous cornstarch vehicle by grinding and mixing with a ground glass homogenizer. Vehicle and the test compound are administered orally in a volume of 10 ml/kg once daily for 3 days beginning 1 hour prior to intrapleural injection of carrageenan. Twenty-four hours after the last dose the rats are sacrificed and their pleural cells collected and counted. Differential counts are made as needed.

In this assay, the percent of reduction in cellular infiltration into the pleural cavity of the treated rats compared to controls is determined and checked for statistical significance using Sudent's t-test. Statistical analysis of the data, using cellcounts rather than percent change, is performed by using the Brown-Forsythe method.

The experiments are run with 5,6-dihydro-1,4(4H)-thiazin-2-yl, 4-fluorophenyl ketone as representative test compound with 11 rats per group at a dose of 25 mg/kg p.o. and 12 rats per group at a dose of 50 mg/kg.

The compounds of this invention are also active in the decrease of neutrophil adherence assay described in Amer. J. Med. *61*, 597 (1976).

The advantageous pharmacological properties render the compounds of the present invention useful as disease-modifying antirheumatic agents especially for the treatment and amelioration of e.g. rheumatic disorders, such as rheumatoid arthritis in mammals, including man.

The pharmaceutical compositions according to the invention are those suitable for enteral, such as oral or rectal, parenteral such as subcutaneoous or intravenous, or topical administration to mammals, including man, for the treatment of rheumatic diseases such as rheumatoid arthritis comprising an affective amount of a pharmacologically active compound of formula I, or a pharmaceutically acceptable salt thereof, exclusively or in combination with one or more pharmaceutically acceptable adjuvants or carriers.

The pharmacologically active compounds of the invention are useful in the manufacture of pharmaceutical compositions containing an effective amount thereof in conjunction or admixture with excipients suitable for either enteral, parenteral or topical application. Preferred are tablets and gelatin capsules comprising the active ingredient together with a) diluents, e.g. lactose, dextrose, sucrose, mannitol, sorbitol, cellulose and/or glycine) b) lubricants, e.g. silica, talcum, stearic acid, its magnesium or calcium salt and/or polyethyleneglycol; for tablets also c) binders, e.g. magnesium aluminium silicate, starch paste, gelatine, traganth, methylcellulose, sodium carboxymethylcellulose and/or polyvinyl/pyrrolidone; if desired, d) disintegrants, e.g. starches, agar, alginic acid or its sodium salt, or effervescent mixtures; and/or e) absorbents, colorants, flavors and sweeteners. Injectable compositions are preferably aqueous isotonic solutions or suspensions and suppositories. Topical lotions are advantageously made from fatty emulsions or suspensions. They may be sterilized and/or contain adjuvants, such as preserving, stabilizing, wetting or emulsifying agents, solution promoters, salts for regulating the osmotic pressure and/or buffers. Said pharmaceutical compositions may also contain other therapeutically valuable substances. They are prepared according to conventional mixing, granulating or coating methods respectively and contain about 0.1 to 75%, preferably about 1 to 50%, of the active ingredient.

A unit dosage for a mammal of about 50 to 70 kg may contain between about 10 to 200 mg of the active ingredient.

The compounds of formula I, wherein $R^1$ is hydrogen, are prepared by the following processes, preferably by

a) cyclizing a compound of the formula II,

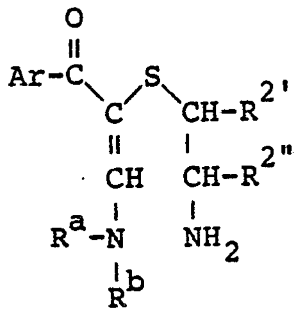

II

wherein Ar is defined as above, $R^a$ and $R^b$ are $C_{1-4}$-alkyl, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxy-carbonyl-$C_{1-4}$-alkyl, and wherein the amino group is in the free form or is protected by a conventional amino protecting group, in the presence of a base or

b) condensing a compound of the formula III,

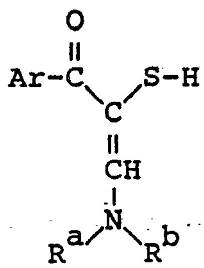

III

wherein Ar and $R^a$ and $R^b$ are defined as above and wherein the mercapto group is in the free form or is protected by a conventional mercapto protecting group with a compound of the formula IV,

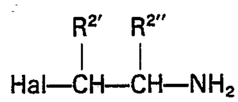

IV

wherein Hal is chloro, bromo or iodo and $R^{2'}$ and $R^{2''}$ are defined as above, or with an acid addition salt thereof in the presence of base and if desired, converting a resulting compound into another compound of the invention and/or converting a resulting salt into the free compound or into a different salt and/or converting a resulting free compound having a salt forming group into a salt.

*Process a)*: In a compound of the formula II, $R^a$ and $R^b$ are especially methyl, and $R^{2'}$ and $R^{2''}$ are especially hydrogen.

The protection of the amino group by a conventional protecting group is described in the references mentioned above with respect to the protection of the carboxy group.

The amino group can, for example, be protected in the form of an acylamino or, preferably, in the form of an acylimido group.

In an acylamino group, acyl is, for example, the acyl group of an organic carboxylic acid having up to 10 carbon atoms, especially of an unsubstituted lower alkanecarboxylic acid, or of a lower alkanecarboxylic acid that is substituted, for example, by halogen or aryl, or of a benzoic acid that is unsubstituted or substituted, for example, by halogen, lower alkoxy or nitro, or of a carbonic acid semi-ester. Such an acyl group is, for example, 2,2,2-trifluoro- or 2,2,2-trichloroacetyl, benzoyl, or benzoyl substituted, for example, by halogen, for example chlorine, lower alkoxy, for example methoxy, or by nitro, for example benzoyl, 4-chlorobenzoyl, 4-methoxybenzoyl, or 4-nitrobenzoyl, or lowr alkoxycarbonyl that is branched in the 1-position of the lower alkyl radical or is substituted in the 1- or 2-position by suitable substituents.

Lower alkoxycarbonyl branched in the 1-position of the lower alkyl radical is, for example, tert-lower alkoxycarbonyl, for example tert-butoxycarbonyl (BOC), arylmethoxycarbonyl having one or two aryl radicals wherein aryl is preferably phenyl that is unsubstituted or mono-, di- or tri-substituted, for example, by lower alkyl, especially tert-lower alkyl, for example tert-butyl, lowr alkoxy, for example methoxy, hydroxy, halogen, for example chlorine, and/or by nitro, for example diphenylmethoxycarbonyl or di-(4-methoxyphenyl)methoxycarbonyl.

Lower alkoxycarbonyl substituted in the 1- or 2-position by suitable substituents is, for example, aroylmethoxycarbonyl, for example phenacyloxycarbonyl, 2-halo-alkoxycarbonyl, for example 2,2,2-trichloroethoxycarbonyl, 2-bromoethoxycarbonyl or 2-iodoethoxycarbonyl, or 2-(tri-substituted silyl)-ethoxycarbonyl in which the silyl group is substituted by organic radicals, for example lower alkyl, phenyl-lower alkyl or phenyl, for example 2-tri-lower alkylsilylethoxycarbonyl, for example 2-trimethylsilylethoxycarbonyl or 2-(di-n-butyl-methylsilyl)-ethoxycarbonyl, or 2-triphenylsilylethoxycarbonyl.

In an acylamido group acyl is preferably the acyl group of a dicarboxylic acid, for example succinic, glutaric or phthalic acid.

The amino group preferably is protected in the form of a succinimido or phthalimido group.

A suitable base is a primary amine, preferably a lower alkylamine, for example n-butylamine, or a secondary amine, for example a di-lower alkylamine, for example diisopropylamine.

The cyclisation is generally carried out in organic inert solvents, such as suitable alcohols, such as methanol, ethanol or isopropanol, ketones, such as acetone, ethers, such as dioxan or tetrahydrofuran, nitriles, such as acetonitrile, halogenated hydrocarbons, such as methylene chloride, chloroform or carbon tetrachloride, esters, such as ethyl acetate, or amides, such as dimethylformamide or dimethylacetamide, and the like. The reaction temperature is between room temperature and the boiling temperature of the reaction mixture, preferably between 60°C and the boiling temperature of the reaction mixture.

The cyclisation is preferably carried out under inert gas atmosphere, preferably nitrogen atmosphere.

*Process b)*: In a compound of formula III, $R^a$ and $R^b$ are especially methyl.

The protection of the mercapto group by a conventional protecting group is described in the reference mentioned above with respect to the protection of the carboxy group. The mercapto group can, for example, be protected in the form of an acylthio group.

In an acylthio group acyl is, for example, halo-substituted lower alkanoyl, for example 2,2-dichloroacetyl, or, especially, the acyl radical of a carbonic acid semiester mentioned above in connection with protected amino groups, for example, 2,2,2-trichloroethoxycarbonyl, or 4-nitrobenzyloxycarbonyl, or, preferably, lowr alkanoyl, for example formyl or acetyl. The mercapto group preferably is protected by the formyl or acetyl group.

A suitable base is an inorganic base, for example an alkali metal or alkaline earth metal hydroxide, carbonate or bicarbonate, for example sodium, potassium or calcium hydroxide, carbonate or bicarbonate, or preferably an alkali metal alcoholate, for example sodium or potassium methylate, ethylate or tert-butylate.

The condensation of a compound of the formula III with a compound of the formula IV is preferably carried out in an inert, preferably anhydrous, solvent or solvent mixture, for example in a carboxylic acid amide, for example a formamide, for example dimethylformamide, a halogenated hydrocarbon, for example methylene chloride, carbon tetrachloride or chlorobenzene, a ketone, for example acetone, a cyclic ether, for example tetrahydrofuran, an ester, for example ethyl acetate, or a nitrile, for example acetonitrile, or in mixtures thereof, preferably in an alcohol, for example methanol or ethanol, or water, optionally at reduced or elevated temperature, for example in a temperature range from approximately −40°C to approximately +100°C, preferably from approximately −10°C to room temperature, and optionally under an inert gas atmosphere, for example nitrogen atmosphere.

The starting materials employed in the process for the preparation of compounds of the formula I are known or, if they are novel, can be obtained in a manner known per se.

Starting materials of the formula II are novel and are also subject matter of the present invention. They can be prepared by reacting a compound of the formula V,

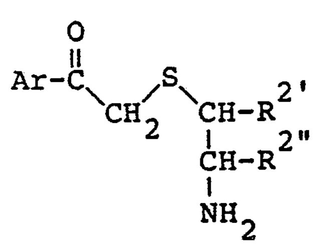

V

wherein Ar, $R^{2'}$ and $R^{2''}$ are defined as above and wherein the amino group is protected by a conventional amino protecting group, with a reactive, functional derivative of N,N-di-$C_{1-4}$-alkylformamide. A conventional amino protecting group is mentioned above under process a) and is, preferably, phthaloyl. A

reactive, functional derivative of N,N-di-$C_{1-4}$-alkylformamide, for example dimethylformamide, is, for example, a formiminium salt of the formula VIa

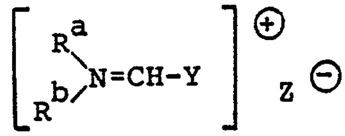

VIa

wherein $R^a$ and $R^b$ are lower alkyl, for example ethyl, preferably methyl, Y is halogen, for example bromine, or, preferably, chlorine, lower alkoxy, for example ethoxy, or, preferably, methoxy, or benzyloxy, and Z is halogen, preferably chlorine, lower alkoxy, the radical of an alkylating agent, for example monomethylsulfate, orthodimethylphosphate, or the tetrafluoroborate radical; or is a formamide acetal of the formula VIb,

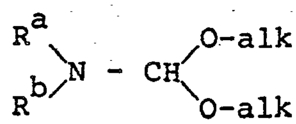

VIb .

wherein $R^a$ and $R^b$ are defined as above and alk is methyl or ethyl.

The formiminium salt of the formula VIa, wherein Y and Z are halogen, can be obtained by reacting a N,N-di-$C_{1-4}$-alkylformamide, for example dimethylformamide with a halogenating agent, for example oxalyl chloride, phosgene, diphosgene (trichloromethyl chloroformate), phosphoryl chloride, phosphorous pentachloride or thionyl chloride. Compounds of the formula VIa, wherein Y is lower alkoxy, preferably methoxy, and Z is the radical of an alkylating agent, for example the monomethylsulfate radical or the tetrafluoroborate radical, are obtained by reacting the N,N-di-$C_{1-4}$-alkylformamide with an alkylating agent, for example a lower alkyl halide, for example methyl iodide, or a lower alkylsulfate, for example dimethylsulfate, or by reaction with a tri-lower alkyloxonium salt, for example trimethyloxoniumtetrafluoroborate. These reactions are carried out in an inert, dry solvent, especially in methylene chloride, chloroform, diethylether, benzene or toluene at temperatures between about −10° and the boiling point of the reaction mixture. The resulting formiminium salt can be isolated and purified or, as described above, produced and used further in situ.

The formamide acetal of the formula VIb can be obtained by reacting an alcohol, such as a lower alkanol, for example methanol or ethanol, with an iminium ether salt of the formula VIa described above (Y = lower alkoxy or benzyloxy), preferably in the presence of basic agents, for example alcoholates, for example sodium methylate, or by treating a dialkylamine, for example dimethylamine, with an activated or reactive derivative or orthoformic acid such as an ester or an amide acetal thereof, for example with an orthoester of formic acid, preferably in the presence of Lewis catalysts, for example with trimethyl orthoformiate and zinc chloride or boron trifluoride etherate or by trating a N,N-di-$C_{1-4}$-alkylformamide, for example dimethylformamide, in a manner known per se with an acetalizing agent, such as a trialkyloxonium tetrafluoroborate, for example triethyloxonium tetrafluoroborate, and, if desired, subsequently treating the reaction product with a basic agent for example with sodium methylate.

The reaction conditions are known and their choice depends in particular on the vigor of the reaction to be expected. Thus, the reaction of an iminium ether salt with sodium methylate or an alcohol in the presence of a tertiary base is preferably carried out with cooling, for example at temperatures of about −70°C to about +10°C. However, it is also possible to carry out the reaction at higher temperatures, i.e. for example, of up to about 75°C, if the stability of the starting materials and of the reaction products permits a higher temperature.

The process variant which proceeds via the starting materials of the formula VIa is advantageously carried out in an inert organic solvent; the process variant which proceeds via the starting compounds of the formula VIb using orthoformiates as the condensing agent preferably proceeds in the presence of an alkylating catalyst, such as a Lewis acid, for example zinc chloride or boron trifluoride etherate.

Suitable inert solvents or diluents are dry, preferably polar, solvents which do not possess any reactive functional groups. In particular, halogenated hydrocarbons, especially methylene chloride or chloroform, and also ketones, such as acetone, ethers, such as diethyl ether, anisole or tetrahydrofuran, and also aromatic hydrocarbons, for example benzene or toluene, or esters which are stable to hydrolysis, such as ethyl acetate, ae suitable as solvents.

Compounds of the formula II can also be prepared by reacting a compound of the formula VII

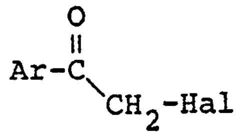

VII

wherein Ar is defined as above and Hal is bromine or iodine, preferably chlorine, with an ethylene episulfide compound of the formula

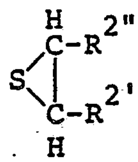

wherein $R^{2'}$ and $R^{2''}$ are defined as above, reacting the salt obtained of the formula

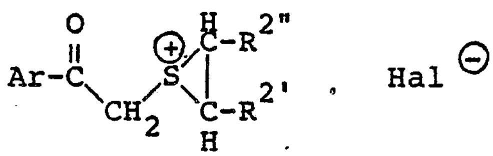

with the formamide acetal of the formula VIb to give a compound of the formula

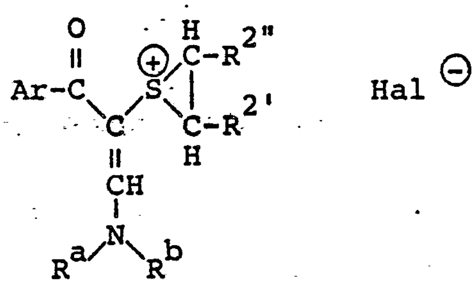

which is reacted with ammonia to give a compound of the formula II.

Starting materials of the formula III are novel and can be prepared by reacting a compound of the formula VIII

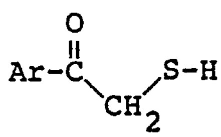

VIII

wherein Ar is defined as above and wherein the mercapto group is protected by a conventional mercapto protecting group, with a reactive, functional derivative of N,N-di-$C_{1-4}$-alkylformamide.

A conventional mercapto protecting group is mentioned above and is preferably acetyl. A reactive, functional derivative of N,N-di-$C_{1-4}$-alkylformamide is, for example, a formiminium salt of the formula VIa or a formamide acetal of the formula VIb. The process is carried out in a manner analogous to the process for the preparation of compounds of the formula V.

Starting materials of the formula IV are known. They are commercially available or can be prepared by known methods.

Starting materials of the formula V are novel and can be prepared by condensing a mercaptane of the formula IX,

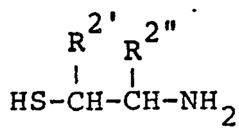

IX

wherein $R^{2'}$ and $R^{2''}$ are defined as above and wherein the amino group is protected by a conventional amino protecting group, under intermediary protection of the mercapto group with a conventional mercapto protecting group with a halide of the formula VII,

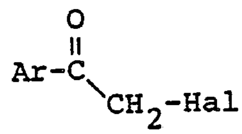

VII

wherein Ar is defined as above and Hal is bromine or iodine, preferably chlorine, in the presence of a base.

A conventional amino protecting group is mentioned above under Process a) and is, preferably, phthaloyl. A conventional mercapto protecting group is mentioned above and is preferably acetyl. This protecting group is split off during the process. This process can be carried out with the same base, preferably sodium methoxide, in the same solvent, e.g. ethanol, and under the same reaction conditions mentioned above under process b).

Compounds of formula VIa and VIb are known. Their preparation is described in Houben-Weyl, Methoden der Organischen Chemie, Thieme Verlag, Stuttgart 1965, Sauerstoff-Verbindungen I, Teil 3, pg. 199—367.

Starting materials of the formula IX are known or, if they are novel, can be prepared by condensing a compound of the formula IV with a compound of the formula X,

$$R_o—S—H \qquad X$$

wherein $R_o$ is a leaving group, preferably acetyl, in the presence of a base. This process can be carried out with the same base, preferably sodium methoxide, in the same solvent, e.g. ethanol, and under the same reaction conditions mentioned above under process b).

Compounds of the formulae VII, VIII, IX and X are known and are either commercially available or can be prepared according to known methods.

Furthermore, the compounds of the invention can be prepared by

1. cyclizing a compound of the formula XI,

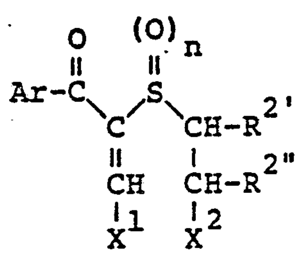

XI

wherein Ar and n have the meaning as defined above, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl, one of $X^1$ and $X^2$ represents a leaving group and the other of $X^1$ and $X^2$ represents $NHR^1$ wherein $R^1$ has meaning as defined above, or

2. condensing a compound of the formula XII,

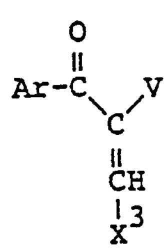

XII

with a compound of the formula XIII,

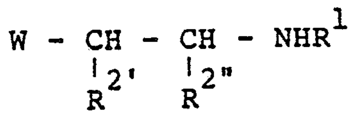

XIII

wherein in a compound of formula XII Ar has meaning as defined hereinabove and $X^3$ represents a leaving group, in a compound of formula XIII $R^1$, $R^{2'}$ and $R^{2''}$ have meaning as defined hereinabove; and in compounds of formula XII and XIII one of V and W represents a leaving group and the other of V and W represents mercapto; or

3. condensing a compound of the formula XIV,

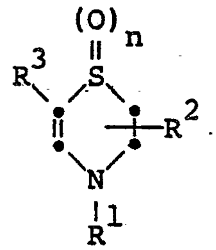

XIV

wherein $R^1$, $R^2$ and n have meaning as defined above, and $R^3$ represents carboxy or functionalized carboxy, with a compound of the formula Ar-M wherein Ar has meaning as defined hereinabove and M represents alkali metal or halomagnesium; or

4. reducing a compound of the formula XV,

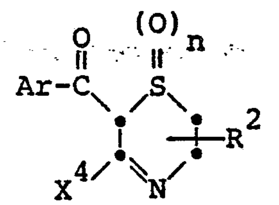

XV

wherein Ar, $R^2$ and n have meaning as defined hereinabove, $X^4$ represents hydroxy or a leaving group, under elimination conditions to obtain a compound of formula I wherein $R^1$ represents hydrogen; or

5. eliminating $HX^5$ from a compound of the formula XVI,

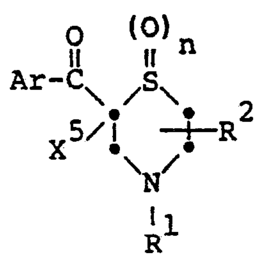

XVI

wherein Ar, $R^1$, $R^2$ and n have meaning as defined hereinabove and $X^5$ represents a leaving group; or

6. cyclizing a compound of the formula XVII,

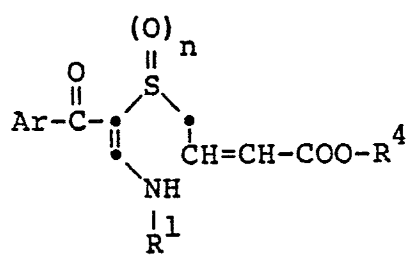

XVII

wherein Ar, $R^1$ and n have meaning as defined hereinabove and $R^4$ represents $C_{1-4}$-alkyl; to obtain a compound of the formula I wherein $R^2$ represents $C_{1-4}$-alkoxycarbonylmethyl; or

7. saturating the $R^2$-substituted double bond in a compound of the formula XVIII,

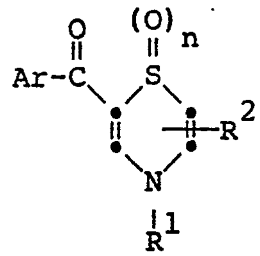

XVIII

wherein Ar, $R^1$, $R^2$ and n have a meaning as defined hereinabove; and when required temporarily protecting in any of above processes any interfering reactive functional groups; and if desired, converting a resulting compound into another compound of the invention and/or converting a resulting salt into the free compound or into a different salt and/or converting a resulting free compound having a salt forming group into a salt.

Process 1

The cyclization is carried out e.g. as described for process a) hereinabove.

A leaving group, as represented by $X^1$ or $X^2$ includes a reactive esterified hydroxy group, such as halo (e.g. chloro, bromo or iodo), lower alkylsulfonyloxy (e.g. methanesulfonyloxy) or arylsulfonyloxy (e.g. p-toluenesulfonyloxy); or a di-lower alkylamino group (e.g. dimethylamino).

The starting materials are prepared using conventional reactions. For example, the starting materials of structure XI, e.g. those of formula II, are prepared, preferably in situ, as described hereinabove. Other starting materials of structure XI, e.g. wherein $X^1$ represents halo, are prepared, preferably in situ, e.g. by first treating a compound of the formula $ArCOCH{=}CH{-}OH$ with a halogen, e.g. bromine, in an inert solvent such as chloroform, followed by thionyl chloride to yield a compound of the formula

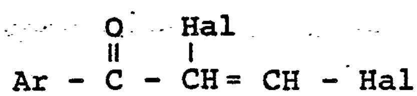

in which Hal represents halo, e.g. bromo, and reacting said intermediate with a compound of formula IXa,

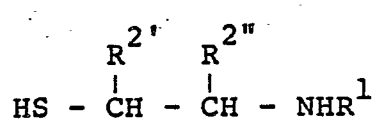

IXa

wherein $R^1$, $R^{2'}$ and $R^{2''}$ have meaning as defined for compounds of formula IX to obtain a compound of formula XI wherein $X^1$ represents halo and $X^2$ represents $NHR^1$.

Process 2

The condensation is carried out e.g. under conditions described for process b) above.

A leaving group as represented by V or W is preferably a reactive esterified hydroxy group as defined above. A leaving group as represented by $X^3$ is preferably halogen or di-lower alkylamino, e.g. dimethylamino.

The starting materials of formula XII and XIII are prepared e.g. as described for compounds of formula III and IV above or conventional modifications thereof.

Process 3

The condensation is preferably carried out in an inert atmosphere, e.g. under nitrogen, at a low temperature, e.g. ranging from about −70° to +25°C in an anhydrous solvent, such as an alicyclic or cyclic ether, e.g. diethyl ether or tetrahydrofuran. Functionalized carboxy $R^3$ is preferably cyano, a tertiary amide- or an ester-grouping, such as a di-lower alkyl amide- or a substituted or unsubstituted lower alkyl ester-grouping. The starting materials of formula XIV are either known or may be prepared according to methods described in the literature, e.g. Tetrahedron *24*, 2985 (1968). The reagent Ar-M, e.g. the compound wherein M represents lithium, is either known or prepared according to methods well known in the art.

Process 4

The reduction according to process 4 is carried out with e.g. an alkali metal borohydride reducing agent such as sodium borohydride in a polar solvent such as a lower alkanol at a temperature ranging from about −25° - to +50°C. A leaving group as represented by $X^4$ is preferably halogen or lower alkoxy. Elimination of $HX^4$ occurs simultaneously or on workup.

The starting materials of formula XV can be prepared by first reacting an appropriately substituted thiomorpholin-3-one with a compound of the formula ArCOOH, or a reactive derivative thereof, such as a lower alkyl ester or an acid chloride, or with a compound of the formula Ar-CN in the presence of a strong base, e.g. a lower alkyl lithium, an alkali metal hydride or amide in an inert solvent, such as ether or tetrahydrofuran at a temperature ranging from about −70° to +50°C; and then treating the resulting 2-ArCO-substituted-thiomorpholin-3-one with a halogenating agent, e.g. phosphorus oxychloride, to yield a compound of formula XV wherein $X^4$ represents halo, or with e.g. a trilower alkyloxonium fluoroborate to yield a compound of formula XV wherein $X^4$ represents a lower alkoxy.

Process 5

The elimination of $HX^5$ according to process 5, (advantageously for the preparation of compounds wherein n=O) can be carried out, e.g., with heat in a polar solvent such as glacial acetic acid.

The leaving group $X^5$ is preferably halo or lower alkanoyloxy.

The starting material of formula XVI can be prepared in situ from the corresponding 2-ArCO-substituted-2,3,5,6-tetrahydro-1-oxothiazine under conditions of a Pummerer rearrangement, e.g. with acetic anhydride.

The 2-ArCO-substituted-2,3,5,6-tetrahydro-1-oxothiazine is in turn prepared by condensation of a compound of the formula

$$\begin{array}{c} \qquad\quad Br \\ \qquad\quad | \\ Ar-CO-C=CH_2 \end{array}$$

with a compound of formula IX (or amino protected form thereof), deprotection if required, and oxidation of the 2-ArCO-substituted-2,3,5,6-tetrahydrothiazine so obtained with e.g. hydrogen peroxide.

Process 6

The cyclization according to process 6 is preferably carried out in the presence of a base, such as an alkali metal lower alkoxide, e.g. sodium methoxide, in a polar solvent such as ethanol or dimethylformamide at a temperature of about 25°—100°C.

A starting compound of formula XVII can be prepared by first condensing a compound of formula III with e.g. a $C_{1-4}$-alkyl ester of 4-halocrotonic acid, and then condensing the resulting product with a compound of the formula $R^1NH_2$ wherein $R^1$ has meaning as defined hereinabove.

Process 7

The saturation of the double bond according to process 7 may be carried out with a reducing agent such as sodium borohydride.

The compounds of the invention, so obtained, can be converted into each other according to methods known per se. For example, if $R^1$ is hydrogen, the secondary amino group can be acylated to give compounds wherein $R^1$ is $C_{1-4}$-alkanoyl. The acylation can be effected by reaction with a suitable acylating agent which introduces the $C_{1-4}$-alkanoyl radical, for example formic or acetic acid, or a reactive functional derivative thereof.

A reactive, functional derivative of a carboxylic acid is an anhydride of this carboxylic acid or a mixed anhydride. A mixed anhydride is formed e.g. by condensation with another acid, e.g. an inorganic acid such as a hydrohalic acid, and is, for example, the corresponding carboxylic acid halide, e.g. the carboxylic acid chloride or bromide. A reactive functional derivative of a carboxylic acid of the formula III is furthermore formed by condensation with a lowr alkyl hemiester of carbonic acid, e.g. the ethyl or isobutyl hemiester of carbonic acid.

The acylation reactions are preferably carried out in the presence of a suitable acid acceptor, for example of a suitable organic base. A suitable organic base is e.g. an amine, e.g. a tertiary amine such as a tri-lower alkylamine, e.g. trimethylamine or triethylamine, a cyclic tertiary amine such as N-methylmorpholine, a bicyclic amidine, e.g. a diazabicycloalkene such as 1,5-diazabicyclo[4.3.0]non-5-ene or 1,5-diazabicyclo[5.4.0]undec-5-ene (DBU), or is, for example, a base of the pyridine type, e.g. pyridine. A suitable acid acceptor is also an inorganic base, for example an alkali metal hydroxide or alkaline earth metal hydroxide, e.g. sodium, potassium or calcium hydroxide.

The acylation reactions are preferably carried out in an inert, preferably anhydrous, solvent or mixture of solvents, for example in dimethylformamide, methylene chloride, carbon tetrachloride, chlorobenzene, acetone, tetrahydrofuran, ethyl acetate or acetonitrile, or in mixtures thereof, if desired at low or elevated temperature, e.g. in the temperature range from about −40°C to +100°C, preferably from about −10° to +50°C, and optionally in an inert gas amtosphere, e.g. under nitrogen.

If in a compound of formula I $R^1$ is hydrogen, the secondary amino group can be alkylated with a suitable alkylating agent that introduces the $C_{1-4}$-alkyl or the heterocyclyl-$C_{2-4}$-alkyl group to give compounds of formula I, wherein $R^1$ is $C_{1-4}$-alkyl or heterocyclyl-$C_{2-4}$-alkyl. The secondary amino group is treated with a suitable metallating agent, for example sodium or lithium diisopropylamide, butyllithium or

potassium hydride, and the metallated compound is reacted with an alkylating reagent, for example methyliodide or 2-(piperid-1-yl)-ethylchloride.

If in a compound of the formula I $R^2$ is carboxy-$C_{1-4}$-alkyl, the free carboxy group can be converted into a carboxy group that can be cleaved under physiological conditions, for example enzymatically, by esterification methods known per se.

For example, a compound of the formula I in which the carboxy group to be esterified is in the free form or a compound of the formula I in which the carboxy group to be esterified is in the form of a reactive, functional derivative, for example in the form of an acid chloride, or a salt of a compound of the formula I is reacted with the corresponding alcohol or with a reactive functional derivative of this alcohol, for example the halide, e.g. chloride.

If in a compound of the formula I $R^2$ is $C_{1-4}$-alkoxycarbonyl-$C_{1-4}$-alkyl, the ester can be converted to the free carboxylic acid of formula I, wherein $R^2$ is carboxy-$C_{1-4}$-alkyl, for example by known ester cleavage methods, for example saponification under acid conditions, for example by reaction with hydrochloric acid or sulfuric acid, or by reaction with sodium or potassium hydroxide and converting the salt obtained to the free acid.

A compound of the formula I wherein n is zero, can be converted to a compound of the formula I wherein n is one, by adding one equivalent of an oxidizing agent. Such oxidizing agents are especially hydrogen peroxide or organic peracids, especially aliphatic percarboxylic acids, for example peracetic acid, perbenzoic acid, m-chloroperbenzoic acid, or monoperphthalic acid.

A compound of the formula I wherein n is zero or one, can be converted to a compound of the formula I wherein n is two, by reaction with two equivalents or an excess or one equivalent of the oxidizing agents mentioned above.

The oxidation is preferably carried out in a suitable non-aqueous inert solvent, for example a halogenated hydrocarbon, for example methylene chloride, chloroform or carbon tetrachloride, an alcohol, for example methanol or ethanol, a ketone, for example acetone, an amide, for example dimethylformamide, or a liquid organic carboxylic acid, for example acetic acid, or in a mixture of these solvents, at room temperature, or while cooling or gently heating, for example from approximately −50°C to approximately +40°C, preferably from approximately −20°C to approximately 0°C. The oxidation can also be carried out in stages by first oxidising at low temperature, that is to say from approximately −20°C to approximately −10°C, to the sulfoxide stage ($n = 1$), which is optionally isolated, and then in a second stage, by oxidizing the sulfoxide to the sulfone ($n = 2$), preferably at higher temperature, for example from −10° to 0°C.

For working up, excess oxidizing agent which may still be present can be eliminated by reaction with a reducing agent, for example a thiosulfate, for example sodium thiosulfate.

A 1-oxide of the formula I in which *n* is 1, and a 1,1-dioxide in which *n* is 2, can be converted by various reducing agents, for example phosphorus trichloride, into the corresponding 1-sulfide in which *n* is 0.

Salts of compounds of the formula I can be manufactured in a manner known per se. Thus, salts of compounds of the formula I can be prepared by reaction of a compound having a carboxy group with aqueous solutions of alkaline metal hydroxides, such as sodium or potassium hydroxide or with ammonia or a suitable organic amine, preferably stoichiometric quantities or only a small excess of the salt-forming agent being used. Acid addition salts of compounds of the formula I wherein $R^1$ is hydrogen are obtained in the customary manner, for example by treatment with an acid or a suitable anion exchange reagent. Salts can be converted into the free compounds in customary manner; metal and ammonium salts can be converted, for example, by treatment with suitable acids, and acid addition salts can be converted, for example, by treatment with a suitable basic agent.

The invention also includes those partial embodiments according to which compounds formed as intermediates are used as starting materials and the remaining process steps are carried out with these, or the process is discontinued at any stage; furthermore, starting materials may be used in the form of derivatives or may be formed during the reaction.

Preferably, the starting materials and the reaction conditions are so chosen that the compounds described above as being especially preferred are obtained.

The following examples are intended to illustrate the invention and are not to be construed as being limitations thereon. Temperatures throughout are given in degrees Centigrade and all parts wherever given are parts by weight. If not otherwise stated, evaporations are carried out under reduced pressure, preferably between about 2 and 13 kPa, IR-spectra in $cm^{-1}$.

## Example 1

2.527 kg n-Butylamine is added to a stirred solution of 23 l ethanol and 4.58 kg 2 - [2 - (2 - dimethylamino - 1 - (4 - fluorobenzoyl)vinylthio)ethyl] - 1,3 - isoindoledione under nitrogen atmosphere and the solution is heated at reflux temperature for 24 hours. The reaction mixture is cooled and stirred at 10° for 3 hours. The crude product is collected by filtration, washed several times with ethanol and ether and dried. The crude product is dissolved at 90° in dimethylformamide and the solution is filtered through a heated funnel. A solid begins to form and the resulting suspension is stirred at room temperature overnight, filtered, washed with ether and dried to give 5,6-dihydro-1,4(4H)-thiazin-2-yl-4-fluorophenyl ketone, m.p. 212—214°, IR (N): NH 3100, C=O 1580.

The starting materials are prepared as follows:

a) 0.855 kg Potassium-tert-butoxide is added in portions at −5° under nitrogen atmosphere to 4 l dimethylformamide. A solution of 0.6 kg thioacetic acid in 2 l dimethylformamide is added in a steady steam at such a rate that the temperature can be maintained between −5° and 0°. The reaction mixture is then stirred for 1 hour at −5°. 2.0 kg N-(2-bromoethyl)phthalimide in 4 l dimethylformamide is added to the reaction mixture at such a rate that the temperature does not exceed 5° and the reaction mixture is stirred for 2 hours. 40 l of water cooled at 5—10° are added which raises the reaction temperature to 20°. The reaction mixture is stirred overnight at room temperature and filtered. The crude product is washed with water and dried to yield 2-(2-acetylthioethyl)-1,3-isoindoledione, m.p. 112—114°.

b) 0.803 kg sodium methoxide in 16 l of ethanol is added to a stirred solution of 3.7 kg 2-(2-acetylthioethyl)-1,3-isoindoledione in 29.2 l ethanol at 0°. The temperature is maintained between 0° and 5° and the reaction mixture is stirred for one hour. 3.23 kg α-bromo-4-fluoroacetophenone in 14.6 l of ethanol is added at such a rate to maintain the temperature between 0° and 10°.The reaction mixture is stirred overnight at room temperature. The product is collected by filtration, washed with ethanol and ether and dried to yield 2-(2-(4-fluorobenzoylmethylthio)ethyl)-1,3-isoindoledione, m.p. 123.5—125.5°.

c) 1.525 kg of dimethylformamide dimethylacetal is added to a solution of 4.4 kg 2-(2-(4-fluorobenzoylmethylthio)ethyl)-1,3-isoindoledione in 44 l dichloromethane. The solution is heated at reflux temperature for 24 hours, another 0.76 kg of the acetal are added and the solution is heated under reflux for another 24 hours. The solvent is removed at reduced temperatures and the heavy oil residue is dissolved in isopropanol at 70°. A solid is formed after stirring overnight which is collected by filtration, washed with ether and dried to yield 2 - [2 - (2 - dimethylamino - 1 - (4 - fluorobenzoyl)vinylthio) - ethyl] - 1,3 - isoindoledione, m.p. 108—111°, IR (N): NH 3200, C=O 1610.

Example 2

To a solution of 39.56 g α-acetylthio-β-dimethylaminoacrylophenone in 300 ml absolute ethanol stirring under nitrogen at 0° a solution of 8.64 g sodium methoxide in 230 ml ethanol is added dropwise over a period of 15 minutes. After complete addition, the dark red solution is stirred at 0 to −3° for 0.5 hours. To the solution stirring at −3° a solution of 32.79 g 2-bromoethylamine hydrobromide in 270 ml absolute ethanol is added dropwise over a period of 25 minutes. After complete addition the dark solution is stirred at 0° for 0.5 hours, and a solution of 8.64 g sodium methoxide in 230 ml absolute ethanol is added dropwise over a period of 15 minutes. The dark solution is stirred at 0° for 1 hour. The reaction mixture is allowed to warm slowly to 20°. A suspension is formed within an hour of removing the cooling bath. The reaction mixture is stirred for 16 hours at ca 20°. The suspension is cooled to 5° and acidified to pH 2 with 30 ml of a 1:1 mixture of ethanol and concentrated hydrochloric acid. The suspension is concentrated in vacuo to remove all but ca 100 ml of ethanol. The residue is suspended in 200 ml chloroform and the mixture is washed with water and a saturated sodium chloride solution. The organic layer is concentrated in vacuo to give a partially crystalline residue. This residue is triturated with a 1:1 mixture of ether and ethanol. Orange crystals are collected to give 5,6-dihydro-1,4(4H)-thiazin-2-yl-phenyl-ketone, m.p. 184—186.5.

The starting materials are prepared as follows:

a) To a solution of 54.0 g sodium methoxide in 100 ml absolute ethanol stirring under nitrogen at reflux temperature 74 ml thioacetic acid is added dropwise over a period of 0.5 hours. Upon complete addition the solution is refluxed for 15 minutes. To the yellow solution stirring under nitrogen atmosphere at reflux temperature, a solution of 199.1 g α-bromoacetophenone in 800 ml absolute ethanol is added rapidly dropwise over a period of 70 minutes. After complete addition, the suspension formed is refluxed for 3 hours. The reaction mixture is cooled and the precipitated sodium bromide is removed by vacuum filtration. The filtrate is concentrated in vacuo to give a brown oil with some solid present. This mixture is suspended in 500 ml of ether and is filtered to remove insoluble sodium bromide. The filtrate is stirred with activated charcoal for 0.5 hours, filtered, and concentrated in vacuo to give a red-brown oil. This oil is purified by vacuum distillation and the fraction is collected at 133—137°/7—27 Pa and characterized by NMR as α-acetylthioacetophenone.

b) 42.5 ml dimethylformamide dimethylacetal is added at 0° dropwise over a period of 17 minutes to 57.0 g α-acetylthioacetophenone stirring under nitrogen. Upon complete addition the orange solution is stirred at 0—5° for ca 4 hours, allowed to warm slowly to room temperature and is stirred overnight. The resulting dark solution is concentrated in vacuo to give a dark oil being α-acetylthio-β-dimethylaminoacrylophenone. The NMR spectrum confirms the presence of the enamine moiety.

Example 3

The following compounds can be prepared by following the experimental procedure according to either one of the examples 1 or 2 or other methods described herein.

a) 5,6-dihydro-1-oxo-1,4(4H)-thiazin-2-yl phenyl ketone, m.p. 198—200°, IR: NH 3100, C=O 1575,

b) 5,6-dihydro-1,1-dioxo-1,4(4H)-thiazin-2-yl phenyl ketone, m.p. 274—276°, IR (N): NH 3180, C=O 1550,

c) 5,6-dihydro-1,4(4H)-thiazin-2-yl 4-chlorophenyl ketone, m.p. 217—219°, IR(N): NH 3200, C=O 1610,

d) 5,6-dihydro-1,4(4H)-thiazin-2-yl 4-bromophenyl ketone, m.p. 243—245°, IR(N) NH 3200, C=O 1600,

e) 5,6-dihydro-1,4(4H)-thiazin-2-yl 3-fluorphenyl ketone, m.p. 191—192°, IR(N): NH 3100, C=O 1600,

f) 5,6-dihydro-1,4(4H)-thiazin-2-yl 3,4-dichlorophenyl ketone, m.p. 222—224°, IR(N): C=O 1605,

g) 5,6-dihydro-1,4(4H)-thiazin-2-yl 2-fluorophenyl ketone, m.p. 205—206°, IR(N): C=O 1602,

h) 5,6-dihydro-1,4(4H)-thiazin-2-yl 4-methoxyphenyl ketone, m.p. 177—178°, IR(N): C=O 1590,

i) 5,6-dihydro-1,4(4H)-thiazin-2-yl 3-chlorophenyl ketone, m.p. 175—176°, IR(N): C=O 1600,

k) 5,6-dihydro-4-(2-piperid-1-yl-ethyl)-1,4(4H)-thiazin-2-yl 3-fluorophenyl ketone, m.p. 169—171°, IR(N): C=O 1560,

l) 5,6-dihydro-4-acetyl-1,4(4H)-thiazin-2-yl 4-fluorophenyl ketone, m.p. 152—153°, IR(N): C=O 1685 and 1630,

m) 5,6-dihydro-1-oxo-1,4(4H)-thiazin-2-yl 3-fluorophenyl ketone, m.p. 218—220°, IR(N): NH 3200, C=O 1560,

n) 5,6-dihydro-1,1-dioxo-1,4(4H)-thiazin-2-yl 3-fluorophenyl ketone, m.p. 263—266°, IR(N): NH 3300—3200, C=O 1550,

o) 5,6-dihydro-1,4(4H)-thiazin-2-yl 3-trifluoromethylphenyl ketone, m.p. 177—179°, IR(N): NH 3200, C=O 1600,

p) 5,6-dihydro-4-methyl-1,4(4H)-thiazin-2-yl 4-fluorophenyl ketone, m.p. 122—123°,

q) 5,6-dihydro-1,4(4H)-thiazin-2-yl pyrid-3-yl-ketone, m.p. 197—199°, IR(N): NH 3250, C=O 1610,

r) 5,6-dihydro-1,4(4H)-thiazin-2-yl 4-carboxy-methylphenyl ketone, m.p. 274—275°, IR(N): NH 3280, C=O 1700 and 1610,

s) 5,6-dihydro-1,4(4H)-thiazin-2-yl 3-carboxy-methylphenylketone, m.p. 254—255°, IR(N): NH 3240, C=O 1695 and 1600,

t) 5-ethoxycarbonylmethyl-5,6-dihydro-1,4(4H)-thiazin-2-yl phenyl ketone, m.p. 143—145°, IR(N): NH 3190, C=O 1725 and 1600,

u) 5-carboxymethyl-5,6-dihydro-1,4(4H)-thiazin-2-yl phenyl ketone, m.p. 251°, IR(N): NH 3200, C=O 1700 and 1605.

Example 4

To 0.1 mole of 5,6-dihydro-2-methoxycarbonyl-1,4(4H)-thiazine [Tetrahedron *24*, 2985 (1968)] at −70° in 100 ml of ether under nitrogen atmosphere is added dropwise 0.2 moles of 4-fluorophenyllithium (prepared from 4-fluorophenyl bromide and n-butyl lithium) in 100 ml of ether at such a rate as to maintain a temperature of −70°. The resulting mixture is allowed to stir for 2 hours at −70° and then allowed to warm to room temperature. Careful acidification with 1N hydrochloric acid is followed by extraction with chloroform. After drying, filtration and removal of chloroform there is obtained 5,6-dihydro-1,4(4H)-thiazin-2-yl 4-fluorophenyl ketone (example 1, m.p. 212—214°).

Similarly 5,6-dihydro-1,4(4H)-thiazine-2-carboxylic acid (using 3 mole equivalents of 4-fluorophenyl lithium), 5,6-dihydro-2-cyano-1,4(4H)-thiazine or 5,6-dihydro-2-dimethylcarbamoyl-1,4(4H)-thiazine may be condensed with 4-fluorophenyl lithium to yield 5,6-dihydro-1,4(4H)-thiazin-2-yl 4-fluorophenyl ketone.

Example 5

a) To a suspension of 5.65 g of 2-aminoethanethiol hydrochloride, 20.24 g of triethylamine and 100 ml ethanol stirring at 5° is added a solution of 12.60 g of α,β-dibromoacrylophenone in 25 ml of ethanol dropwise at such a rate so as to maintain the reaction temperature below 10°. Upon complete addition the suspension is stirred at 5—10° for one hour then at room temperature for 1 hour. The reaction mixture is then heated slowly to reflux (complete solution effected at 45°) and maintained at reflux for ca 16 hours. The solution is cooled in an ice bath and the solid which forms is collected and discarded. The filtrate is diluted with diethyl ether whereupon more solid forms which is filtered off. The filtrate is concentrated in vacuo. The residue is suspended in 200 ml of 7% sodium bicarbonate solution and extracted with ethyl acetate (2 × 200 ml). The combined organic extracts are washed with 1N hydrochloric acid (1 × 200 ml) and saturated sodium chloride solution (1 × 200 ml). The organic layer is decolorized with activated charcoal, filtered, dried over magnesium sulfate, filtered and concentrated in vacuo to give a residue which is triturated with ethyl acetate to give 5,6-dihydro-1,4(4H)-thiazin-2-yl phenyl ketone of example 2.

The starting material is prepared as follows:

To a suspension of 65.2 g of the sodium salt of benzoylacetaldehyde in 500 ml chloroform stirring at room temperature is added 60.7 g bromine dropwise; during the addition the reaction temperature rises to approximately 50°. Upon complete addition the reaction mixture is stirred without heating until room temperature is reached. To the reaction mixture is added 45.2 g of thionyl chloride dropwise. Upon complete addition the reaction mixture is stirred overnight at room temperature. The insoluble material is removed by vacuum filtration and the filtrate concentrated in vacuo to give an oil which is distilled under reduced pressure (ca 400 Pa) to give α,β-dibromoacrylophenone; NMR: all protons between 7.2 and 8.18.

b) 5,6-Dihydro-1,4-(4H)-thiazin-2-yl 4-fluorophenyl ketone of example 1 is similarly prepared starting with 4-fluorobenzoylacetaldehyde in the first reaction.

Example 6

a) To a solution of 3.40 g 2-benzoyl-thiomorpholin-3-one in 40 ml methylene chloride stirring at room temperature is added 15 ml of 1.0M solution of triethyloxonium tetrafluoroborate in methylene chloride. Upon complete addition the solution is washed with one 5 ml portion of 10% sodium hydroxide solution

and one 5 ml portion of saturated sodium chloride solution. The organic layer is dried over magnesium sulfate, filtered, and concentrated in vacuo to give 3-ethoxy-5,6-dihydro-1,4(2H)-thiazin-2-yl phenyl ketone, m.p. 112—114°.

To a solution of 1.00 g of the iminoether in 10 ml ethanol while stirring under nitrogen at −5° is added 0.15 g sodium borohydride in one portion. The reaction mixture is stirred at room temperature for a few and thin layer chromatography (silica gel, chloroform/methanol 9:1) of the resulting product gives 5,6-dihydro-1,4(4H)-thiazin-2-yl phenyl ketone, the compound of example 2.

The starting material is prepared as follows:

To a suspension of 35.15 g of thiomorpholin-3-one and 1.5 l tetrahydrofuran while stirring under nitrogen at −70°, is added 305 ml of a 1.97M solution of n-butyl lithium. Upon complete addition the solution is stirred under nitrogen at −70° for 1 hour. To the reaction mixture is added 45.05 g of ethyl benzoate dropwise. Upon complete addition the reaction mixture is stirred at −70° for 4 hours and then at room temperature overnight. To the reaction mixture is added 200 ml of water and 350 ml of 3N hydrochloric acid. The organic layer is separated and the aqueous layer extracted with ether (2 × 600 ml). The combined organic layers are washed with saturated sodium chloride solution (300 ml), dried over magnesium sulfate, filtered and concentrated in vacuo. The residue is treated with a small amount of ether, the solid is collected and recrystallized from methanol to give 2-benzoyl-thiomorpholin-3-one, m.p. 110—114°.

b) 5,6-Dihydro-1,4(4H)-thiazin-2-yl 4-fluorophenyl ketone is similarly prepared starting with ethyl 4-fluorobenzoate in the first reaction.

Example 7

To a mixture of 3.35 g of 5,6-dihydro-1,4(4H)-thiazin-2-yl 4-fluoropenyl ketone in 50 ml of 95% ethanol is added 1.70 g of 30% hydrogen peroxide. This mixture is heated at reflux temperature for 2 hours. The solution is concentrated to a yellow solid which is recrystallized from ethanol to give 5,6-dihydro-1-oxo-1,4(4H)-thiazin-2-yl 4-fluorophenyl ketone, m.p. 220—222°.

Example 8

To a suspension of 0.01 mole of 5,6-dihydro-1-oxo-1,4(4H)-thiazin-2-yl 4-fluorophenyl ketone and 0.024 mole of sodium iodide in 50 ml of acetone is added a solution of 0.017 mole of trifluoroacetic anhydride in 30 ml of acetone. After stirring for 1 hour the solution is filtred to remove inorganic material. The acetone filtrate is concentrated in vacuo and the residue dissolved in chloroform. This solution is washed with sodium thiosulfate solution, then brine, dried, and concentrated in vacuo to give 5,6-dihydro-1,4(4H)-thiazin-2-yl 4-fluorophenyl ketone of example 1.

Example 9

A mixture of 3.35 g of 5,6-dihydro-1,4(4H)-thiazin-2-yl 4-fluorophenyl ketone, 17 ml of acetic anhydride, and 3.64 ml of pyridine is heated at 130—145° for 2.5 hours. After cooling the solution is poured over an ice-water mixture. The resulting precipitate is collected, washed with water and dried to give 5,6-dihydro-4-acetyl-1,4(4H)-thiazin-2-yl 4-fluorophenyl ketone, m.p. 152—153°, of example 3 l.

Example 10

a) A mixture of 0.01 mole of 5,6-dihydro-4-acetyl-1,4(4H)-thiazin-2-yl 4-fluorophenyl ketone in 50 ml of 3N hydrochloric acid is heated at 100° for 1 hour. The solution is cooled, the resulting precipitate is filtered off, washed and dried to yield 5,6-dihydro-1,4(4H)-thiazin-2-yl 4-fluorophenyl ketone of example 1.

b) Treatment of 5,6-dihydro-4-acetyl-1,4(4H)-thiazin-2-yl 4-fluorophenyl ketone with 2N sodium hydroxide in 50% aqueous ethanol at 50° yields 5,6-dihydro-1,4(4H)-thiazin-2-yl 4-fluorophenyl ketone.

Example 11

a) To 1.2 g of a 50% dispersion of sodium hydride in mineral oil in 30 ml of dimethylformamide is added dropwise at room temperature under nitrogen a solution of 6.2 g of 5,6-dihydro-1,4(4H)-thiazin-2-yl 4-fluorophenyl ketone in 120 ml of dimethylformamide. The mixture is stirred at room temperature for 1 hour, then cooled to 0—5° with an ice-water bath. To this cooled suspension is added 3.9 g of methyl iodide and this mixture is stirred for 4 hours. At this time the pH of the mixture is adjusted to ca 4 with 3N hydrochloric acid followed by addition of a saturated sodium bicarbonate solution. The product is extracted with 2 × 200 ml of methylene chloride. The organic extract is washed with 2 × 300 ml water, dried and concentrated to an oil. The solid obtained after trituration with petroleum ether is recrystallized from ethanol-cyclohexane to give 5,6-dihydro-4-methyl-1,4(4H)-thiazin-2-yl 4-fluorophenyl ketone, m.p. 122—123°, of example 3p.

b) Similarly, treatment of 5,6-dihydro-1,4(4H)-thiazin-2-yl 4-fluorophenyl ketone with piperidinoethyl chloride yields 5,6-dihydro-4-(2-piperid-1-yl-ethyl)-1,4(4H)-thiazin-2-yl 4-fluorophenyl ketone, m.p. 169—171° of example 3k.

Example 12

a) To an ice-cooled suspension of 7.50 g of 5,6-dihydro-1,4(4H)-thiazin-2-yl phenyl ketone in 38 ml of

glacial acetic acid while stirring is added 25% peracetic acid in 28.30 g of ethyl acetate dropwise. After complete addition the suspension is stirred at 0—2° for 1 hour. The cooling bath is then removed and the suspension stirred without external cooling at room temperature for about 22 hours. An additional 11.3 g of 25% peracetic acid in ethyl acetate is added dropwise at room temperature. Upon complete addition stirring is continued without external heating or cooling for about 22 hours. The precipitated product is collected, washed with water and methanol, and dried. A suspension of the product in boiling methanol is filtered to yield 5,6-dihydro-1,1-dioxo-1,4(4H)-thiazin-2-yl phenyl ketone of example 3b.

b) Similarly prepared is 5,6-dihydro-1,1-dioxo-1,4(4H)-thiazin-2-yl 4-fluorophenyl ketone, m.p. 288—290°.

Example 13

To a solution of 1.90 g of α-[(3-ethoxycarbonyl-2-propenyl)-thiol-β-aminoacrylophenone in 30 ml absolute ethanol stirring under nitrogen at 27° is added dropwise a solution of 0.35 g of sodium methoxide in absolute ethanol. After completion addition the solution is stirred at room temperature for 2 hours and at reflux for 2.5 hours. The reaction mixture is acidified to pH 2 with a 1:1 mixture of ethanol and concentrated hydrochloric acid. The reaction mixture is filtered and the filtrate concentrated in vacuo. The residue is dissolved in 50 ml chloroform and the solution washed with water. the chloroform extract is dried over magnesium sulfate, filtered, and concentrated in vacuo. The residue is recrystallized from ethyl acetate to give 5-ethoxycarbonylmethyl-5,6-dihydro-1,4(4H)-thiazin-2-yl phenyl ketone of example 3t.

The starting material is prepared as follows:

To a solution of 29.9 g of α-acetylthio-β-dimethylaminoacrylophenone in 250 ml of absolute ethanol stirring under nitrogen at 0—5° is added a solution of 6.48 g of sodium methoxide in 150 ml absolute ethanol dropwise over a period of 20 minutes. The resulting solution is stirred at 0° for 1 hour. To the reaction mixture stirring at 0° is added 35.00 g of methyl 4-bromocrotonate (85%) dropwise over a period of 5 minutes. After complete addition, the reaction mixture is stirred at 0° for 15 minutes. The cooling bath is removed and the reaction is allowed to warm to room temperature. The reaction mixture is stirred at room temperature for ca 1.5 hour, then placed in a freezer for 2 days. The sodium bromide is removed by filtration and the filtrate is concentrated in vacuo. The residue is dissolved in ether, the solution is washed with water and saturated sodium chloride solution, dried over magnesium sulfate, filtered, and concentrated in vacuo to give crude α - [(3 - ethoxycarbonyl - 2 - propenyl)thiol] - β - dimethylaminoacrylophenone.

A mixture of 32.55 g of this crude product, 76.31 g of ammonium acetate and 500 ml of absolute ethanol is stirred and heated at reflux under nitrogen for 2.5 hours. The solution is concentrated in vacuo. The residue is suspended in 60 ml 50% aqueous ethanol and the suspension is concentrated in vacuo. The material is dissolved in 250 ml ethyl acetate and the solution washed with water, and saturated sodium chloride solution. The organic layer is stirred with activated charcoal, dried over magnesium sulfate, filtered, and evaporated to dryness. The residue is chromatographed over silica gel eluting with chloroform-methanol (97:3) to give α - [(3 - ethoxycarbonyl - 2 - propenyl)thio] - β - aminoacrylophenone.

Example 14

A suspension of 1.30 g of 5 - ethoxycarbonylmethyl - 5,6 - dihydro - 1,4(4H) - thiazin - 2 - yl phenylketone in 80 ml hydrochloric acid is stirred and heated at 75—80° for 4 hours. The suspension is cooled to 3°, and the solid collected, washed with water and dried to give 5 - carboxymethyl - 5,6 - dihydro - 1,4(4H) - thiazin - 2 - yl phenyl ketone of example 3u.

Example 15

a) A suspension of 10.00 g of α-bromo-β-dimethylaminoacrylophenone hydrobromide, 3.41 g of 2-aminoethanethiol hydrochloride, 3.04 g of triethylamine and 200 ml ethanol is stirred at room temperature for 45 minutes, then heated to reflux. The resulting solution is stirred and refluxed for 18 hours. An additional 3.0 g of triethylamine is added and refluxing continued for 5 hours. The solution is concentrated in vacuo. The residue is dissolved in 50 ml 1N hydrochloric acid solution and extracted with ethyl acetate (4 × 100 ml). The organic extract is washed with a saturated sodium chloride solution (2 × 100 ml), dried over magnesium sulfate, filtered, and concentrated in vacuo to give a partially crystalline residue. This residue is triturated with 200 ml of a 1:1 mixture of diethyl ether/ethanol. The resulting solid is collected to give 5,6-dihydro-1,4(4H)-thiazin-2-yl phenyl ketone, the compound of example 2.

The starting material is prepared as follows:

A mixture of 14.42 g acetophenone and 17.67 g of dimethylformamide diethyl acetal is stirred and heated at 100—105° for ca. 18 hours. The reaction mixture is cooled to room temperature and the solid formed is collected and washed with ethanol/diethyl ether to give β-dimethylaminoacrylophenone, m.p. 90—92°. To a solution of 17.06 g of β-dimethylaminoacrylophenone in 170 ml of Chloroform stirring at room temperature is added a solution of 16.30 g bromine in 30 ml of chloroform dropwise. During the addition a thick suspension forms. Upon complete addition the reaction mixture is stirred at room temperature for 1.5 hours. The solid is collected, washed with chloroform and recrystallized from ethanol/diethylether to give α-bromo-β-dimethylaminoacrylophenone hydrobromide, m.p. 1422° (dec.).

b) Similarly prepared is 5,6-dihydro-1,4(4H)-thiazin-2-yl 4-fluorophenyl ketone of example 1, starting with p-fluoroacetophenone.

Example 16

2,3,5,6-Tetrahydro-1-oxo-1,4(4H)-thiazin-2-yl 4-fluorophenyl ketone is reacted with 1 mole equivalent of acetic anhydride is glacial acetic acid at 100° to yield 5,6-dihydro-1,4(4H)-thiazin-2-yl 4-fluoro-phenyl ketone of example 1.

The starting material is prepared as follows:

A solution of α-bromo-p-fluoroacrylophenone (prepared according to Nippon Kagaku Zasshi *84*, 650) in ethanol is treated with a mole equivalent of 2-phthalimidoethanethiol in the presence of sodium ethoxide to yield α-(2-phthalimidoethylthio)-p-fluoroacrylophenone. Reaction with n-butylamine (to the free amine) and cyclization in situ gives 2,3,5,6-tetrahydro-1,4(4H)-thiazin-2-yl 4-fluorophenyl ketone. Treatment with hydrogen peroxides as described in example 7 yields 2,3,5,6-tetrahydro-1-oxo-1,4-(4H)-thiazin-2-yl 4-fluorophenyl ketone.

Example 17

A suspension of 3.5 g of 2 - [2 - (2 - dimethylamino - 1 - (4 - fluorobenzoyl)vinylsulfinyl) - ethyl] - 1,3 - isoindoledione in 100 ml of ethanol is heated at reflux temperature for 24 hours with 2.5 g of *n*-butylamine. The reaction mixture is then cooled to 10°, stirred for 1 hour and the product is collected to yield 5,6-dihydro-1-oxo-1,4(4H)-thiazin-2-yl 4-fluorophenyl ketone of example 7.

The starting material is prepared as follows:

To a mixture of 4.58 g of 2 - [2 - (2 - dimethylamino - 1 - (4 - fluorobenzoyl)vinylthio)ethyl] - 1,3 - isoindoledione from example 1 in 60 ml of 95% ethanol is added 1.70 g of 30% hydrogen peroxide. This mixture is heated at reflux for 2 hours. The reaction mixture is concentrated to give 2 - [2 - (2 - dimethylamino - 1 - (4 - fluorobenzoyl) - vinylsulfinyl) - ethyl] - 1,3 - isoindoledione which is used directly in the next step above.

Example 18

A suspension of 3.7 g of 2 - [2 - (2 - dimethylamino - 1 - (4 - fluorobenzoyl)vinylsulfonyl)ethyl] - 1,3 - isoindoledione in 100 ml of ethanol is heated at reflux temperature for 24 hours with 2.5 g of n-butylamine. The reaction mixture is then cooled and the product is collected to yield 5,6-dihydro-1,1-dioxo-1,4(4H)-thiazin-2-yl 4-fluorophenyl ketone of example 12b.

The starting material is prepared as follows:

A suspension of 4.58 g of 2 - [2 - (2 - dimethylamino - 1 - (4 - fluorobenzoyl)vinylthio)ethyl] - 1,3 - isoindoledione from example 1 in 50 ml of glacial acetic acid is treated dropwise at 15° with 10.5 g of 25% peracetic acid in ethyl acetate. After complete addition the suspension is allowed to warm to room temperature and stirred for 20 hours. The suspended solid is collected and washed with water and cold ethanol to yield 2 - [2 - (2 - dimethylamino - 1 - (4 - fluorobenzoyl)vinylsulfonyl)ethyl] - 1,3 - isoindoledione which is used directly in the next step above.

Example 19

Preparation of 10,000 tablets each containing 100 mg of the active ingredient with a composition as follows:

| | |
|---|---|
| 5,6-dihydro-1,4(4H)-thiazin-2-yl 4-fluorophenyl ketone | 1,000.00 g |
| Lactose | 2,535.00 g |
| Corn starch | 125.00 g |
| Polyethylene glycol 6,000 | 150.00 g |
| Talcum powder | 150.00 g |
| Magnesium stearate | 40.00 g |
| Purified water | q.s. |

Procedure: All the powders are passed through a screen with openings of 0.6 mm. Then the drug substance, lactose, talcum, magnesium stearate and half of the starch are mixed in a suitable mixer. The other half of the starch is suspended in 65 ml of water and the suspension added to the boiling solution of the polyethylene glycol in 260 ml of water. The paste formed is added to the powders which are granulated, if necessary, with an additional amount of water. The granulate is dried overnight at 35°, broken on a screen with 1.2 mm openings and compressed into tablets, using concave punches with 10.3 mm diameter, uppers bisected.

**Claims for the contracting States: BE CH DE FR GB IT LI LU NL SE**

1. A compound of the formula I,

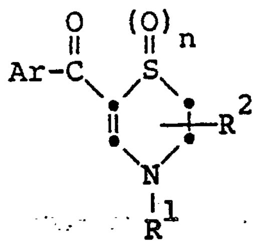

I

wherein Ar is pyridyl, phenyl or phenyl substituted by halogen, halo-$C_{1-4}$-alkyl, $C_{1-4}$-alkoxy or carboxy-$C_{1-4}$-alkyl, n is zero, one or two, $R^1$ is hydrogen, $C_{1-4}$-alkyl, heterocyclyl-$C_{2-4}$-alkyl, amino-$C_{2-4}$-alkyl, $C_{1-4}$-alkylamino-$C_{2-4}$-alkyl, di-$C_{1-4}$-alkylamino-$C_{2-4}$-alkyl or $C_{1-4}$-alkanoyl and $R^2$ is hydrogen, carboxy-$C_{1-4}$-alkyl or $C_{1-4}$-alkoxycarbonyl-$C_{1-4}$-alkyl, with the exception of 5,6-dihydro-1,4(4H)-thiazin-2-yl phenyl ketone and the corresponding sulfoxide and sulfone, and a salt of such compound that has a salt forming group.

2. A compound of formula I according to claim 1, wherein Ar is pyridyl or phenyl substituted by halogen, trifluoromethyl, $C_{1-4}$-alkoxy, or carboxy-$C_{1-4}$-alkyl, n is zero, one or two, $R^1$ is hydrogen, $C_{1-4}$-alkyl, heterocyclyl-$C_{2-4}$-alkyl, di-$C_{1-4}$-alkylamino-$C_{2-4}$-alkyl or $C_{1-4}$-alkanoyl, and $R^2$ is hydrogen or carboxy-$C_{1-4}$-alkyl, and a pharmaceutically acceptable salt of such compound that has a salt forming group.

3. A compound of formula I according to claim 1, wherein Ar represents 3-pyridyl, n is zero, $R^1$ represents hydrogen, $C_{1-4}$-alkyl, or $C_{1-4}$-alkanoyl, and $R^2$ is hydrogen or a compound of formula I, wherein Ar is phenyl monosubstituted by halogen, or phenyl disubstituted, one substituent being trifluoromethyl or halogen, and the other one being halogen, wherein n is zero, one or two, $R^1$ represents hydrogen, $C_{1-4}$-alkyl, or $C_{1-4}$-alkanoyl, and $R^2$ is hydrogen, and a pharmaceutically acceptable salt of such compound that has a salt forming group.

4. A compound of formula I according to claim 1, wherein Ar is phenyl monosubstituted by fluoro or chloro, or phenyl disubstituted, one substituent being trifluoromethyl or fluoro or chloro, and the other one being fluoro or chloro, n is zero, and $R^1$ and $R^2$ are hydrogen.

5. A compound of formula I according to claim 1, wherein Ar is 4-fluorophenyl, n is zero, and $R^1$ and $R^2$ are hydrogen, being 5,6-dihydro-1,4(4H)-thiazin-2-yl 4-fluorophenyl ketone.

6. A pharmaceutical composition containing an antirheumatically effective amount of a compound of the formula I according to claim 1 or a pharmaceutically acceptable salt thereof together with a pharmaceutically acceptable adjunct or carrier.

7. A pharmaceutical composition containing an antirheumatically effective amount of a compound of the formula I, wherein Ar is phenyl, n is zero, one or two and $R^1$ and $R^2$ are hydrogen, together with a pharmaceutically acceptable adjunct or carrier.

8. A compound of formula I according to claim 1 for use in a therapeutic method of treating humans and animals.

9. A compound of the formula I according to claim 1, wherein Ar is phenyl, n is zero, one or two and $R^1$ and $R^2$ are hydrogen, for use in a therapeutic method of treating humans and animals.

10. A compound of formula I according to claim 1 as an antirheumatic.

11. A compound of the formula I according to claim 1, wherein Ar is phenyl, n is zero, one or two and $R^1$ and $R^2$ are hydrogen, as an antirheumatic.

12. The use of a compound of the formula I according to claim 1 in producing of a pharmaceutical composition.

13. The use of a compound of the formula I according to claim 1, wherein Ar is phenyl, n is zero, one or two and $R^1$ and $R^2$ are hydrogen, in producing of a pharmaceutical composition.

14. The use of a compound of the formula I according to claim 1 for the manufacture of a medicament for antirheumatic application.

15. The use of a compound of the formula I according to claim 1, wherein Ar is phenyl, n is zero, one or two and $R^1$ and $R^2$ are hydrogen, for the manufacture of a medicament for antirheumatic application.

16. A compound of the formula II,

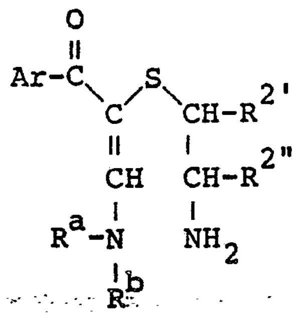

II

wherein Ar is pyridyl or phenyl substituted by halogen, halo-$C_{1-4}$-alkyl, $C_{1-4}$-alkoxy, or carboxy-$C_{1-4}$-alkyl, $R^a$ and $R^b$ are $C_{1-4}$-alkyl, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl.

17. Process for the manufacture of a pharmaceutical composition, wherein a product as claimed in any of claims 1 to 5 is worked up with a pharmaceutical carrier.

18. Process for the manufacture of a pharmaceutical composition, wherein a product as claimed in claim 1, wherein Ar is phenyl, n is zero, one or two and $R^1$ and $R^2$ are hydrogen, is worked up with a pharmaceutical carrier.

19. Process for the manufacture of compounds of formula I shown in claim 1 and salts of these compounds that have a salt forming group which consists in cyclizing a compound of the formula II,

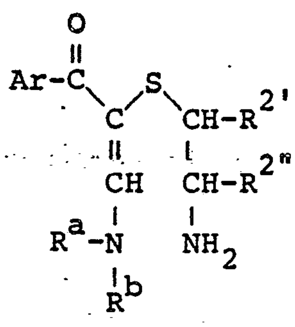

II

wherein Ar is defined as in claim 1, $R^a$ and $R^b$ are $C_{1-4}$-alkyl, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl, and wherein the amino group is in the free form or is protected by a conventional amino protecting group, in the presence of a base, and if desired, converting a resulting compound into another compound of formula I and/or converting a resulting salt into the free compound or into a different salt and/or converting a resulting free compound having a salt forming group into a salt.

20. Process for the manufacture of compounds of formula I shown in claim 1 and salts of these compounds that have a salt forming group which consists in cyclizing a compound of the formula XI,

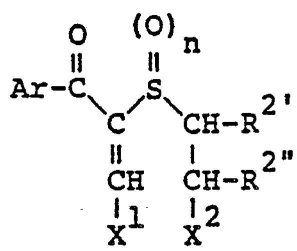

XI

wherein Ar and n have meaning as defined in claim 1, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl, one of $X^1$ and $X^2$ represents a leaving group and the other of $X^1$ and $X^2$ represents $NHR^1$ wherein $R^1$ has meaning as defined in claim 1, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

21. Process for the manufacture of compounds of formula I shown in claim 1 and salts of these compounds that have a salt forming group which consists in condensing a compound of the formula III,

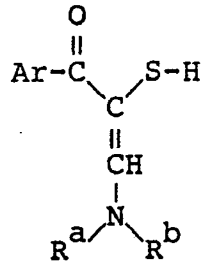

III

wherein Ar and $R^a$ and $R^b$ are defined as in claim 19 and wherein the mercapto group is in the free form or is protected by a conventional mercapto protecting group with a compound of the formula IV,

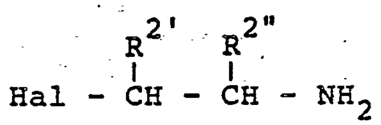

IV

wherein Hal is chloro, bromo or iodo and $R^{2'}$ and $R^{2''}$ are defined as in claim 19, or with an acid addition salt thereof in the presence of base, and if desired, converting a resulting compound into another compound of formula I and/or converting a resulting salt into the free compound or into a different salt and/or converting a resulting free compound having a salt forming group into a salt.

22. Process for the manufacture of compounds of formula I shown in claim 1 and salts of these compounds that have a salt forming group which consists in condensing a compound of the formula XII,

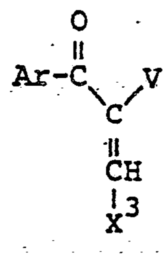

XII

with a compound of the formula XIII,

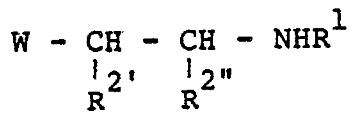

XIII

wherein in a compound of formula XII Ar has meaning as defined in claim 1 and $X^3$ represents a leaving group, in a compound formula XIII, $R^{2'}$ and $R^{2''}$ have meaning as defined in claim 20 and $R^1$ has meaning as defined in claim 1; and in compounds of formula XII and XIII one of V and W represents a leaving group and the other of V and W represents mercapto, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

23. Process for the manufacture of compounds of formula I shown in claim 1 and salts of these compounds that have a salt forming group which consists in condensing a compound of the formula XIV,

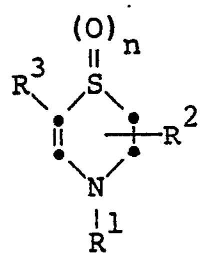

XIV

wherein $R^1$, $R^2$ and n have meaning as defined in claim 1, and $R^3$ represents carboxy or functionalized carboxy, with a compound of the formula Ar-M wherein Ar has meaning as defined in claim 1 and M represents alkali metal or halomagnesium, and when required temporarily protecting any interfering

reactive functional groups; and if desired, converting a resulting compound into another compound of formula I and/or converting a resulting salt into the free compound or into a different salt and/or converting a resulting free compound having a salt forming group into a salt.

24. Process for the manufacture of compounds of formula I shown in claim 1 and salts of these compounds that have a salt forming group which consists in reducing a compound of the formula XV,

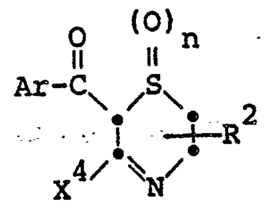

XV

wherein Ar, R, $R^2$ and n have meaning as defined in claim 1 and $X^5$ represents a leaving group, under elimination conditions to obtain a compound of formula I wherein $R^1$ represents hydrogen, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

25. Process for the manufacture of compounds of formula I shown in claim 1 and salts of these compounds that have a salt forming group which consists in eliminating $HX^5$ from a compound of the formula XVI,

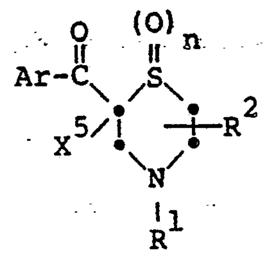

XVI

wherein Ar, $R^1$, $R^2$ and n have meaning as defined in claim 1, $X^5$ represents a leaving group, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

26. Process for the manufacture of compounds of formula I shown in claim 1 and salts of these compounds that have a salt forming group which consists in cyclizing a compound of the formula XVII,

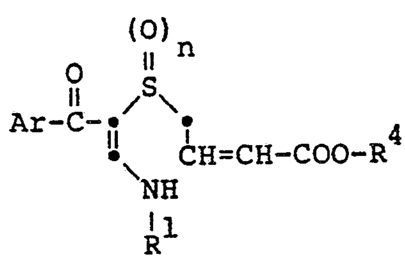

XVII

wherein Ar, $R^1$ and n have meaning as defined in claim 1 and $R^4$ represents $C_{1-4}$-alkyl; to obtain a compound of the formula I wherein $R^2$ represents $C_{1-4}$-alkoxycarbonylmethyl, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

27. Process for the manufacture of compounds of formula I shown in claim 1 and salts of these

compounds that have a salt forming group which consists in saturating the $R^2$-substituted double bond in a compound of the formula XVIII,

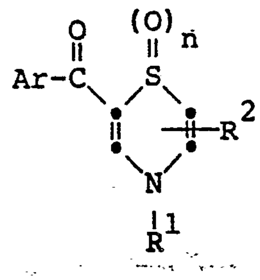

XVIII

wherein Ar, $R^1$, $R^2$ and n have a meaning as defined in claim 1, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

**Claims for the contracting State: AT**

1. A process for the manufacture of a compound of the formula I,

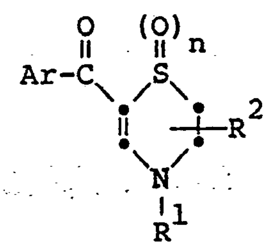

I

wherein Ar is pyridyl, phenyl or phenyl substituted by halogen, halo-$C_{1-4}$-alkyl, $C_{1-4}$-alkoxy or carboxy-$C_{1-4}$-alkyl, n is zero, one or two, $R^1$ is hydrogen, $C_{1-4}$-alkyl, heterocyclyl-$C_{2-4}$-alkyl, amino-$C_{2-4}$-alkyl, $C_{1-4}$-alkylamino-$C_{2-4}$-alkyl, di-$C_{1-4}$-alkylamino-$C_{2-4}$-alkyl or $C_{1-4}$-alkanoyl, and $R^2$ is hydrogen, carboxy-$C_{1-4}$-alkyl or $C_{1-4}$-alkoxycarbonyl-$C_{1-4}$-alkyl, with the exception of 5,6-dihydro-1,4(4H)-thiazin-2-yl phenyl ketone and the corresponding sulfoxide and sulfone, and a salt of such compound that has a salt forming group, which consists in cyclizing a compound of the formula II,

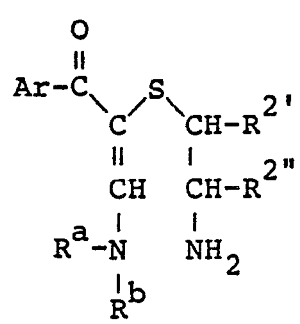

II

wherein Ar is defined as above, $R^a$ and $R^b$ are $C_{1-4}$-alkyl, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl, and wherein the amino group is in the free form or is protected by a conventional amino protecting group, in the presence of a base, and if desired, converting a resulting compound into another compound of formula I and/or converting a resulting salt into the free compound or into a different salt and/or converting a resulting free compound having a salt forming group into a salt.

2. Process for the manufacture of compounds of formula I shown in claim 1 and salts of these compounds that have a salt forming group which consists in condensing a compound of the formula III,

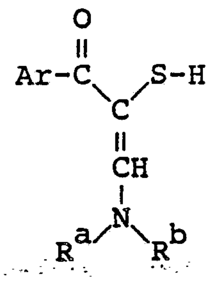

III

wherein Ar and $R^a$ and $R^b$ are defined as in claim 1 and wherein the mercapto group is in the free form or is protected by a conventional mercapto protecting group with a compound of the formula IV,

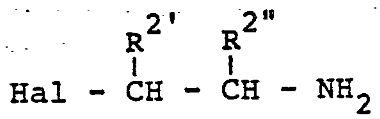

IV

wherein Hal is chloro, bromo or iodo and $R^{2'}$ and $R^{2''}$ are defined as in claim 19, or with an acid addition salt thereof in the presence of base, and if desired, converting a resulting compound into another compound of formula I and/or converting a resulting salt into the free compound or into a different salt and/or converting a resulting free compound having a salt forming group into a salt.

3. A process for the manufacture of compounds of formula I shown in claim 1 and salts of these compounds that have a salt forming group which consists in cyclizing a compound of the formula XI,

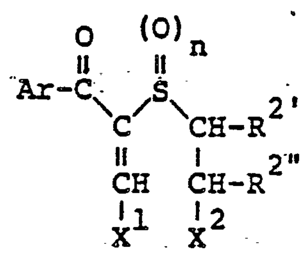

XI

wherein Ar and n have meaning as defined in claim 1, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl, one of $X^1$ and $X^2$ represents a leaving group and the other of $X^1$ and $X^2$ represents $NHR^1$ wherein $R^1$ has meaning as defined above, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

4. A process for the manufacture of compounds of formula I shown in claim 1 and salts of these compounds that have a salt forming group which consists in condensing a compound of the formula XII,

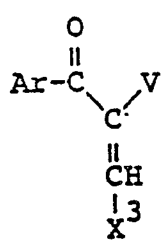

XII

with a compound of the formula XIII,

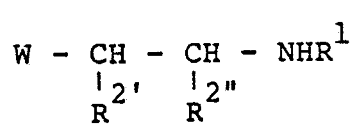

XIII

wherein in a compound of formula XII Ar has meaning as defined in claim 1 and $X^3$ represents a leaving group, in a compound formula XIII $R^{2'}$, and $R^{2''}$ have meaning as defined in claim 1; and in compounds of

formula XII and XIII one of V and W represents a leaving group and the other of V and W represents mercapto, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

5. A process for the manufacture of compounds of formula I shown in claim 1 and salts of these compounds that have a salt forming group which consists in condensing a compound of the formula XIV,

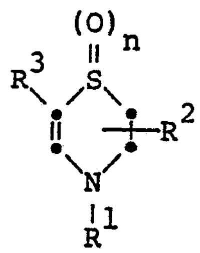

XIV

wherein $R^1$, $R^2$ and n have meaning as defined in claim 1, and $R^3$ represents carboxy or functionalized carboxy, with a compound of the formula Ar-M wherein Ar has meaning as defined in claim 1 and M represents alkali metal or halomagnesium, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

6. A process for the manufacture of compounds of formula I shown in claim 1 and salts of these compounds that have a salt forming group which consists in reducing a compound of the formula XV,

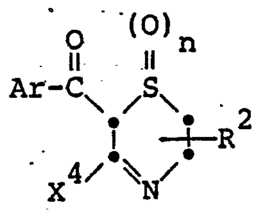

XV

wherein Ar, $R^2$ and n have meaning as defined in claim 1, $X^4$ represents hydroxy or a leaving group, under elimination conditions to obtain a compound of formula I wherein $R^1$ represents hydrogen, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

7. A process for the manufacture of compounds of formula I shown in claim 1 and salts of these compounds that have a salt forming group which consists in eliminating $HX^5$ from a compound of the formula XVI,

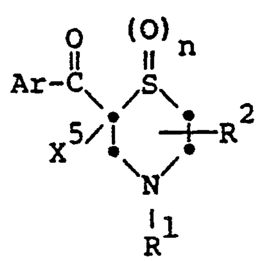

XVI

wherein Ar, $R^1$, $R^2$ and n have meaning as defined in claim 1 and $X^5$ represents a leaving group, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

8. A process for the manufacture of compounds of formula I shown in claim 1 and salts of these compounds that have a salt forming group which consists in cyclizing a compound of the formula XVII,

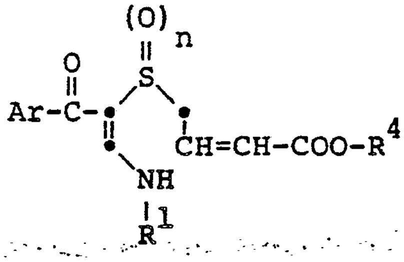

XVII

wherein Ar, $R^1$ and n have meaning as defined in claim 1 and $R^4$ represents $C_{1-4}$-alkyl; to obtain a compound of the formula I wherein $R^2$ represents $C_{1-4}$-alkoxycarbonylmethyl, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

9. A process for the manufacture of compounds of formula I shown in claim 1 and salts of these compounds that have a salt forming group which consists in saturating the $R^2$-substituted double bond in a compound of the formula XVIII,

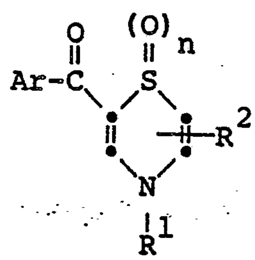

XVIII

wherein Ar, $R^1$, $R^2$ and n have meaning as defined in claim 1, and when required temporarily protecting any interfering reactive functional groups; and if desired, converting a resulting compound into another compound of formula I and/or converting a resulting salt into the free compound or into a different salt and/or converting a resulting free compound having a salt forming group into a salt.

10. A process according to any of claims 1 to 9 for the manufacture of a compound of formula I according to claim I, wherein Ar is pyridyl or phenyl substituted by halogen, trifluoromethyl, $C_{1-4}$-alkoxy, or carboxy-$C_{1-4}$-alkyl, n is zero, one or two, R is hydrogen, $C_{1-4}$-alkyl, heterocyclyl-$C_{2-4}$-alkyl, di-$C_{1-4}$-alkylamino-$C_{2-4}$-alkyl or $C_{1-4}$-alkanoyl, and $R^2$ is hydrogen or carboxy-$C_{1-4}$-alkyl, and a pharmaceutically acceptable salt of such compound that has a salt forming group.

11. A process according to any of claims 1 to 9 for the manufacture of a compound of formula I according to claim 1, wherein Ar represents 3-pyridyl, n is zero, $R^1$ represents hydrogen, $C_{1-4}$-alkyl, or $C_{1-4}$-alkanoyl, and $R^2$ is hydrogen or a compound of formula I, wherein Ar is phenyl monosubstituted by halogen, or phenyl disubstituted, one substituent being trifluoromethyl or halogen, and the other one being halogen, wherein n is zero, one or two, $R^1$ represents hydrogen, $C_{1-4}$-alkyl, or $C_{1-4}$-alkanoyl, and $R^2$ is hydrogen, and a pharmaceutically acceptable salt of such compound that has a salt forming group.

12. A process according to any of claims 1 to 9 for the manufacture of a compound of formula I according to claim 1, wherein Ar is phenyl monosubstituted by fluoro or chloro, or phenyl disubstituted, one substituent being trifluoromethyl or fluoro or chloro, and the other one being fluoro or chloro, n is zero, and $R^1$ and $R^2$ are hydrogen.

13. A process according to any of claims 1 to 9 for the manufacture of a compound of formula I according to claim 1, wherein Ar is 4-fluorophenyl, n is zero, and $R^1$ and $R^2$ are hydrogen, being 5,6-dihydro-1,4(4H)-thiazin-2-yl 4-fluorophenyl ketone.

14. Process for the manufacture of a pharmaceutical composition, wherein a product as claimed in any of claims 1 or 10 to 13 is worked up with a pharmaceutical carrier.

15. Process for the manufacture of a pharmaceutical composition, wherein a product as claimed in claim 1, wherein Ar is phenyl, n is zero, one or two and $R^1$ and $R^2$ are hydrogen, is worked up with a pharmaceutical carrier.

16. A process for the manufacture of a compound of the formula II,

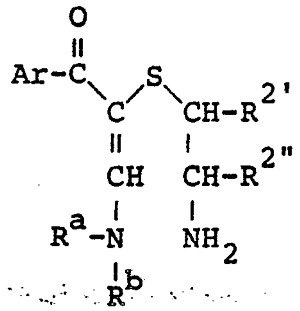

II

wherein Ar is pyridyl or phenyl substituted by halogen, halo-$C_{1-4}$-alkyl, $C_{1-4}$-alkoxy, or carboxy-$C_{1-4}$-alkyl, $R^a$ and $R^b$ are $C_{1-4}$-alkyl, $R^{2'}$ and $R^{2''}$ are hydrogen or one of $R^{2'}$ and $R^{2''}$ is hydrogen and the other is carboxy-$C_{1-4}$-alkyl or $C_{1-4}$-alkoxycarbonyl-$C_{1-4}$-alkyl which consists in reacting a compound of the formula V,

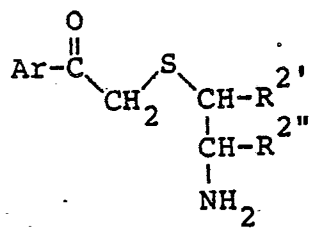

V

wherein Ar, $R^{2'}$ and $R^{2''}$ are defined as in claim 1 and wherein the amino group is protected by a conventional amino protecting group, with a reactive, functional derivative of N,N-di-$C_{1-4}$-alkylformamide.

17. A process for the manufacture of a compound of formula II shown in claim 16 which consists in reacting a compound of the formula VII

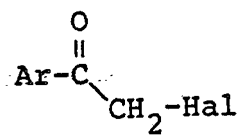

VII

wherein Ar is defined as in claim 1 and Hal is bromine or iodine, preferably chlorine, with an ethylene episulfide compound of the formula

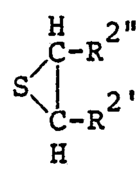

wherein $R^{2'}$ and $R^{2''}$ are defined as in claim 1, reacting the salt obtained of the formula

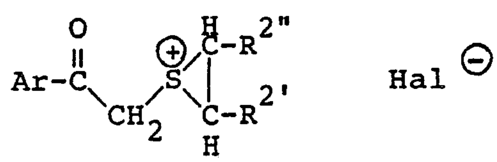

with the formamide acetal of the formula VIb

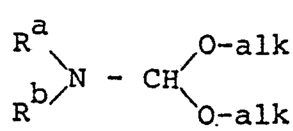

VIb

wherein $R^a$ and $R^b$ are lower alkyl and alk is methyl or ethyl to give a compound of the formula

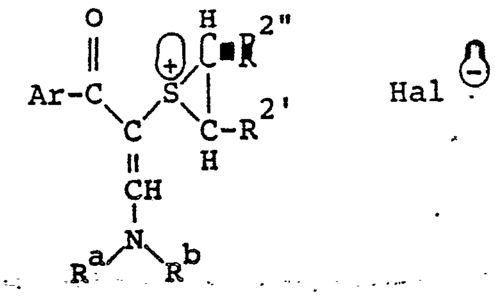

which is reacted with ammonia to give a compound of the formula II.

**Patentansprüche für die Vertragsstaaten: BE CH DE FR GB IT LI LU NL SE**

1. Verbindung der Formel I,

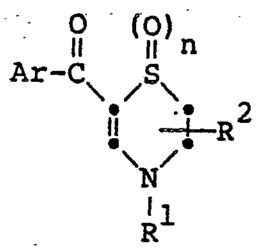

I

worin Ar Pyridyl, Phenyl oder Phenyl, des durch Halogen, Halo-$C_{1-4}$-alkyl, $C_{1-4}$-Alkoxy oder Carboxy-$C_{1-4}$-alkyl substituiert ist, n gleich 0, 1 oder 2 ist, $R^1$ für Wasserstoff, $C_{1-4}$-Alkyl, Heterocyclyl-$C_{2-4}$-alkyl, Amino-$C_{2-4}$-alkyl, $C_{1-4}$-Alkylamino-$C_{2-4}$-alkyl, Di-$C_{1-4}$-alkylamino-$C_{2-4}$-alkyl oder $C_{1-4}$-Alkanoyl steht und $R^2$ für Wasserstoff, Carboxy-$C_{1-4}$-alkyl oder $C_{1-4}$-Alkoxycarbonyl-$C_{1-4}$-alkyl steht, mit der Ausnahme von 5,6-Dihydro-1,4(4H)-thiazin-2-yl-phenyl-keton und dem korrespondierenden Sulfoxid und Sulfon, und ein Salz einer solchen Verbindung, die eine salzbildende Gruppe hat.

2. Verbindung der Formel I nach Anspruch 1, worin Ar Pyridyl oder Phenyl, das substituiert ist durch Halogen, Trifluormethyl, $C_{1-4}$-Alkoxy oder Carboxy-$C_{1-4}$-alkyl ist, n gleich 0, 1 oder 2 ist, $R^1$ für Wasserstoff, $C_{1-4}$-Alkyl, Heterocyclyl-$C_{2-4}$-alkyl, Di-$C_{1-4}$-alkyl-amino-$C_{2-4}$-alkyl oder $C_{1-4}$-Alkanoyl steht und $R^2$ Wasserstoff oder Carboxy-$C_{1-4}$-alkyl ist, und ein pharmazeutisch annehmbares Salz einer solchen Verbindung, die eine salzbildende Gruppe hat.

3. Verbindung der Formel I nach Anspruch 1, worin Ar 3-Pyridyl repräsentiert, n gleich 0 ist $R^1$ für Wasserstoff, $C_{1-4}$-Alkyl oder $C_{1-4}$-Alkanoyl steht, $R^2$ Wasserstoff ist, oder eine Verbindung der Formel I, worin Ar Phenyl ist, das durch Halogen monosubstituiert ist, oder disubstituiertes Phenyl, wobei ein Substituent Trifluormethyl oder Halogen ist, der andere Halogen, worin n gleich 0, 1 oder 2 ist, $R^1$ Wasserstoff, $C_{1-4}$-Alkyl oder $C_{1-4}$-Alkanoyl repräsentiert und $R^2$ Wasserstoff ist, und ein pharmazeutisch annehmbares Salz einer solchen Verbindung, die eine salzbildende Gruppe hat.

4. Verbindung der Formel I nach Anspruch 1, worin Ar durch Fluor oder Chlor monosubstituiertes Phenyl ist oder disubstituiertes Phenyl, wobei ein Substituent Trifluormethyl oder Fluor oder Chlor ist, der andere Fluor oder Chlor, n null ist und $R^1$ und $R^2$ Wasserstoff sind.

5. Verbindung der Formel I nach Anspruch 1, worin Ar 4-Fluorphenyl, n null und $R^1$ und $R^2$ Wasserstoff sind, die 5,6-Dihydro-1,4(4H)-thiazin-2-yl-4-fluorphenyl-keton ist.

6. Pharmazeutische Zusammensetzung, die eine antirheumatisch wirksame Menge einer Verbindung der Formel I nach Anspruch 1 oder ein pharmazeutisch annehmbares Salz davon zusammen mit einem pharmazeutisch annehmbaren Hilfsmittel oder Träger enthält.

7. Pharmazeutische Zusammensetzung, die eine antirheumatisch wirksame Menge einer Verbindung der Formel I enthält, in der Ar Phenyl, n 0, 1 oder 2 ist und $R^1$ und $R^2$ Wasserstoff sind, zusammen mit einem pharmazeutisch annehmbaren Hilfsmittel oder Träger.

8. Verbindung der Formel I nach Anspruch 1 zur Verwendung bei einer therapeutischen Methode der Behandlung von Menschen und Tieren.

9. Verbindung der Formel I nach Anspruch 1, worin Ar Phenyl ist, n gleich 0, 1 oder 2 ist und $R^1$ und $R^2$ Wasserstoff sind, zur Verwendung bei einer therapeutischen Methode der Behandlung von Menschen und Tieren.

10. Verbindung der Formel I nach Anspruch 1 als Antirheumatikum.

11. Verbindung der Formel I nach Anspruch 1, worin Ar Phenyl, n 0, 1 oder 2 ist und $R^1$ und $R^2$ Wasserstoff sind, als Antirheumatikum.

12. Verwendung einer Verbindung der Formel I nach Anspruch 1 bei der Herstellung einer pharmazeutischen Zusammensetzung.

13. Verwendung einer Verbindung der Formel I nach Anspruch 1, worin Ar Phenyl, n gleich 0, 1 oder 2 und $R^1$ und $R^2$ Wasserstoff sind, bei der Herstellung einer pharmazeutischen Zusammensetzung.

14. Verwendung einer Verbindung der Formel I nach Anspruch 1 zur Herstellung eines Medikaments für antirheumatische Anwendung.

15. Verwendung einer Verbindung der Formel I nach Anspruch 1, worin Ar Phenyl ist, n gleich 0, 1 oder 2 ist und $R^1$ und $R^2$ Wasserstoff sind, zur Herstellung eines Medikaments zur antirheumatischen Anwendung.

16. Verbindung der Formel II

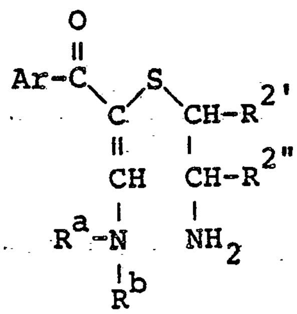

II

worin Ar Pyridyl ist oder Phenyl, das substituiert ist durch Halogen, Halo-$C_{1-4}$-alkyl, $C_{1-4}$-Alkoxy oder Carboxy-$C_{1-4}$-alkyl, $R^a$ und $R^b$ $C_{1-4}$-Alkyl sind, $R^{2'}$ und $R^{2''}$ Wasserstoff sind oder eines von $R^{2'}$ und $R^{2''}$ Wasserstoff ist und das andere Carboxy-$C_{1-4}$-alkyl oder $C_{1-4}$-Alkoxycarbonyl-$C_{1-4}$-alkyl.

17. Verfahren zur Herstellung einer pharmazeutischen Zusammensetzung, worin ein Produkt, wie es in einem der Ansprüche 1 bis 5 beansprucht wird, mit einem pharmazeutischen Träger aufgearbeitet wird.

18. Verfahren zur Herstellung einer pharmazeutischen Zusammensetzung, worin ein Produkt, wie es in Anspruch 1 beansprucht wird, worin Ar Phenyl ist, n gleich 0, 1 oder 2 ist und $R^1$ und $R^2$ Wasserstoff sind, mit einem pharmazeutischen Träger aufgearbeitet wird.

19. Verfahren zur Herstellung von Verbindungen der Formel I — gezeigt im Anspruch 1 — und Salzen dieser Verbindungen, die eine salzbildende Gruppe haben, bestehend im Cyclisieren einer Verbindung der Formel II,

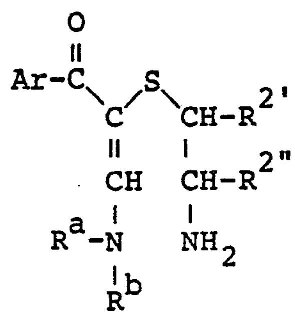

II

worin Ar wie im Anspruch 1 definiert ist, $R^a$ und $R^b$ $C_{1-4}$-Alkyl sind, $R^{2'}$ und $R^{2''}$ Wasserstoff sind oder eines von $R^{2'}$ und $R^{2''}$ Wasserstoff ist und das andere Carboxy-$C_{1-4}$-alkyl oder $C_{1-4}$-Alkoxycarbonyl-$C_{1-4}$-alkyl und worin die Aminogruppe in der freien Form vorliegt oder durch eine herkömmliche Schutzgruppe für Amino geschützt ist, in Gegenwart einer Base und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

20. Verfahren zur Herstellung von Verbindungen der Formel I — gezeigt im Anspruch 1 — und Salzen

dieser Verbindungen, die eine salzbildende Gruppe haben, bestehend im Cyclisieren einer Verbindung der Formel XI,

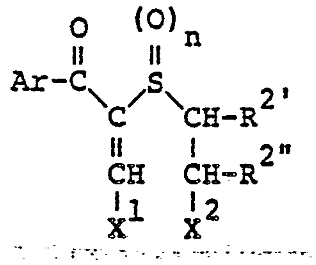

XI

worin Ar und n wie im Anspruch 1 definierte Bedeutung haben, $R^{2'}$ und $R^{2''}$ Wasserstoff sind oder eines von $R^{2'}$ und $R^{2''}$ Wasserstoff ist, das andere Carboxy-$C_{1-4}$-alkyl oder $C_{1-4}$-Alkoxycarbonyl-$C_{1-4}$-alkyl, eines von $X^1$ und $X^2$ eine Abgansgruppe repräsentiert und das andere von $X^1$ und $X^2$ $NHR^1$ repräsentiert, worin $R^1$ die im Anspruch 1 definierte Bedeutung hat, und, wenn erforderlich vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

21. Verfahren zur Herstellung von Verbindungen der Formel I — gezeigt im Anspruch 1 — und Salzen dieser Verbindungen, die eine salzbildende Gruppe haben, bestehend im Kondensieren einer Verbindung der Formel III,

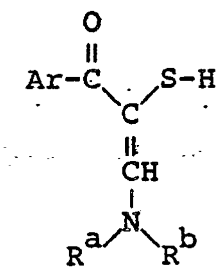

III

worin Ar und $R^a$ und $R^b$ wie im Anspruch 19 definiert sind und worin die Mercaptogruppe in der freien Form vorliegt oder durch eine herkömmliche Mercapto-Schutzgruppe geschützt ist, mit einer Verbindung der Formel IV,

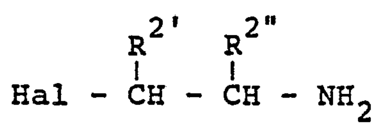

IV

worin Hal Chlor, Brom oder Iod ist, $R^{2'}$ und $R^{2''}$ wie im Anspruch 19 definiert sind, oder mit einem Säureadditionssalz davon, in Gegenwart einer Base, und, falls gewünscht, Umwandeln einer resultierenden Verbindung in eine andere Verbindung der Formel I und/oder Umwandeln eines resultierenden Salzes in die freie Verbindung oder in eine anderes Salz und/oder Umwandeln einer resultierenden freien Verbindung, die eine salzbildende Gruppe hat, in ein Salz.

22. Verfahren zur Herstellung von Verbindungen der Formel I — gezeigt im Anspruch 1 — und Salzen dieser Verbindungen, die eine salzbildende Gruppe haben, bestehend im Kondensieren einer Verbindung der Formel XII,

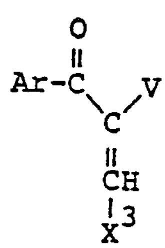

XII

mit einer Verbindung der Formel XIII,

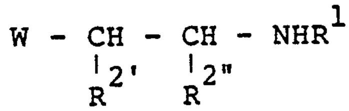

XIII

worin in einer Verbindung der Formel XII Ar die im Anspruch 1 defininierte Bedeutung hat und $X^3$ eine Abgangsgruppe repräsentiert, in einer Verbindung der Formel XIII $R^{2'}$ und $R^{2''}$ die im Anspruch 20 definierte Bedeutung haben, $R^1$ die im Anspruch 1 definierte Bedeutung hat; und in Verbindungen der Formel XII ànd XIII eines von V und W eine Abgangsgruppe repräsentiert und das andere von V und W Mercapto repräsentiert, und, wenn erforderlich, vorübergehenden Schützen störender reaktiver funktioneller Gruppen; und, falls gewünscht, Umwandeln einer restultierenden Verbindung in eine andere Verbindung der Formel I und/oder Unwandeln eines resultierenden Salzes in die freie Verbindung oder in ein anderes Salz und/oder Umwandeln einer resultierenden freien Verbindung, die eine salzbildende Gruppe hat, in ein Salz.

23. Verfahren zur Herstellung von Verbindungen der Formel I — gezeigt im Anspruch 1 — und Salzen jener Verbindungen, die eine salzbildende Gruppe haben, bestehend im Kondensieren einer Verbindung der Formel XIV,

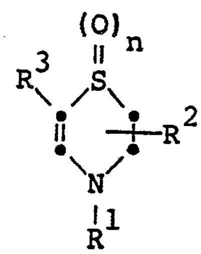

XIV

worin $R^1$, $R^2$ und n die im Anspruch 1 definierte Bedeutung haben, $R^3$ Carboxy oder funktionalisiertes Carboxy repräsentiert, mit einer Verbindung der Formel Ar-M, worin Ar die im Anspruch 1 definierte Bedeutung hat und M Alkalimetall oder Halomagnesium repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

24. Verfahren zur Herstellung von Verbindungen der Formel I — gezeigt im Anspruch 1 — und Salzen jener Verbindungen, die eine salzbildende Gruppe haben, bestehend im Reduzieren einer Verbindung der Formel XV,

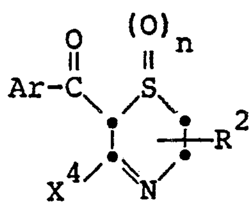

. XV

worin Ar, $R^2$ und n die im Anspruch 1 definierte Bedeutung haben, $X^4$ Hydroxy oder eine Abgangsgruppe repräsentiert, unter Eliminationsbedingungen, um eine Verbindung der Formel I zu erhalten, worin $R^1$ Wasserstoff repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

25. Verfahren zur Herstellung von Verbindungen der Formel I — gezeigt im Anspruch 1 — und Salzen

jener Verbindungen, die eine salzbildende Gruppe haben, bestehend im Eliminieren von $HX^5$ aus einer Verbindung der Formel XVI,

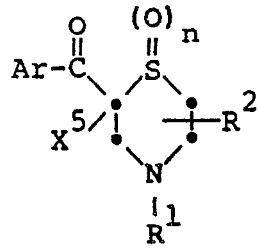

XVI

worin Ar, $R^1$, $R^2$ und n die im Anspruch 1 definierte Bedeutung haben und $X^5$ eine Abgangsgruppe repräsentiert, und, wenn erforderlich, vorübergehenden Schützen störender reaktiver funktioneller Gruppen; und falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

26. Verfahren zur Herstellung von Verbindungen der Formel I — gezeigt im Anspruch 1 — und Salzen dieser Verbindung, die eine salzbildende Gruppe haben, bestehend im Cyclisieren einer Verbindung der Formel XVII,

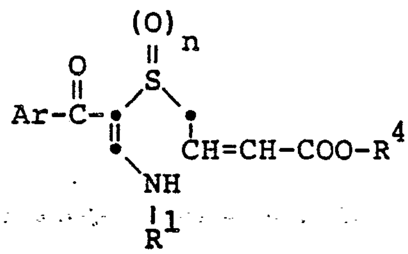

XVII

worin Ar, $R^1$ und n die im Anspruch 1 definierte Bedeutung haben und $R^4$ $C_{1-4}$-Alkyl repräsentiert; um eine Verbindung der Formel I zu erhalten, worin $R^2$ $C_{1-4}$-Alkoxycarbonylmethyl repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

27. Verfahren zur Herstellung von Verbindungen der Formel I — gezeigt im Anspruch 1 — und Salzen jener Verbindungen, die eine salzbildende Gruppe haben, bestehend im Sättigen der $R^2$-substituierten Doppelbindung in einer Verbindung der Formel XVIII,

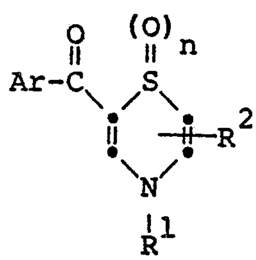

XVIII

worin Ar, $R^1$, $R^2$ und n die im Anspruch 1 definierte Bedeutung haben, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

**Patentansprüche für den Vertragsstaat: AT**

1. Verfahren zur Herstellung einer Verbindung der Formel I

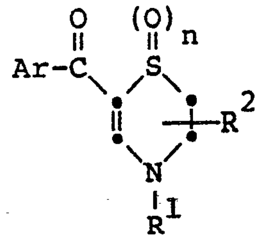

I

worin Ar Pyridyl, Phenyl oder Phenyl, des durch Halogen-$C_{1-4}$-alkyl, $C_{1-4}$-Alkoxy oder Carboxy-$C_{1-4}$-alkyl substituiert ist, n gleich 0, 1 oder 2 ist, $R^1$ für Wasserstoff, $C_{1-4}$-Alkyl, Heterocyclyl-$C_{2-4}$-alkyl, Amino-$C_{2-4}$-alkyl, $C_{1-4}$-Alkylamino-$C_{2-4}$-alkyl, Di-$C_{1-4}$-alkylamino-$C_{2-4}$-alkyl oder $C_{1-4}$-Alkanoyl steht und $R^2$ Wasserstoff, Carboxy-$C_{1-4}$-alkyl oder $C_{1-4}$-Alkoxycarbonyl-$C_{1-4}$-alkyl ist, mit der Ausnahme von 5,6-Dihydro-1,4(4H)-thiazin-2-yl-phenyl-keton und dem korrespondierenden Sulfoxid und Sulfon, und ein Salz einer solchen Verbindung, die eine salzbildende Gruppe hat, bestehend im Cyclisieren einer Verbindung der Formel II,

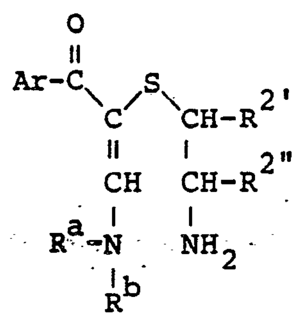

II

worin Ar wie oben definiert ist, $R^a$ und $R^b$ $C_{1-4}$-Alkyl sind, $R^{2'}$ und $R^{2''}$ Wasserstoff sind oder eines von $R^{2'}$ und $R^{2''}$ Wasserstoff ist und das andere Carboxy-$C_{1-4}$-alkyl oder $C_{1-4}$-Alkoxycarbonyl-$C_{1-4}$-alkyl und worin die Aminogruppe in der freien Form vorliegt oder durch eine herkömmliche Schutzgruppe für Amino geschützt ist, in Gegenwart einer Base und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

2. Verfahren zur Herstellung von Verbindungen der Formel I — gezeigt im Anspruch 1 — und Salzen dieser Verbindungen, die eine salzbildende Gruppe haben, bestehend im Kondensieren einer Verbindung der Formel III,

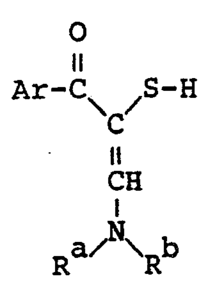

III

worin Ar und $R^a$ und $R^b$ wie im Anspruch 1 definiert sind und worin die Mercaptogruppe in der freien form

vorliegt oder durch eine herkömmliche Mercapto-Schutzgruppe geschützt ist, mit einer Verbindung der Formel IV,

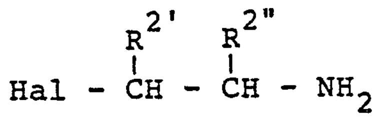

IV

worin Hal Chlor, Brom oder Iod ist, $R^{2'}$ und $R^{2''}$ wie im Anspruch 1 definiert sind, oder mit einem Säureadditionssalz davon, in Gegenwart einer Base, und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die ein salzbildende Gruppe hat, in ein Salz.

3. Verfahren zur Herstellung von Verbindungen der Formel I — gezeigt im Anspruch 1 — und Salzen dieser Verbindungen, die eine salzbildende Gruppe haben, bestehend im Cyclisieren einer Verbindung der Formel XI,

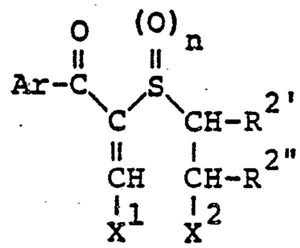

XI

worin Ar und n die im Anspruch 1 definierte Bedeutung haben, $R^{2'}$ und $R^{2''}$ Wasserstoff sind oder eines von $R^{2'}$ und $R^{2''}$ Wasserstoff ist, das andere Carboxy-$C_{1-4}$-alkyl oder $C_{1-4}$-Alkoxycarbonyl-$C_{1-4}$-alkyl, eines von $X^1$ und $X^2$ eine Abgangsgruppe repräsentiert und das andere von $X^1$ und $X^2$ $NHR^1$ repräsentiert, worin $R^1$ die oben definierte Bedeutung hat, und, wenn erforderlich vorübergehenden Schützen störender reaktiver funktionelle Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

4. Verfahren zur Herstellung von Verbindungen der Formel I — gezeigt im Anspruch 1 — und Salzen dieser Verbindungen, die eine salzbildende Gruppe haben, bestehend im Kondensieren einer Verbindung der Formel XII,

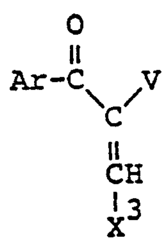

XII

mit einer Verbindung der Formel XIII,

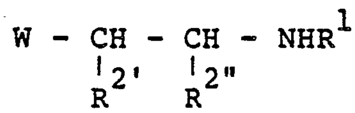

XIII

worin in einer Verbindung der Formel XII Ar die im Anspruch 1 definierte Bedeutung hat und $X^3$ eine Abgangsgruppe repräsentiert, in einer Verbindung der Formel XIII $R^1$, $R^{2'}$ und $R^{2''}$ die im Anspruch 1 definierte Bedeutung haben; und in Verbindungen der Formel XII and XIII eines von V und W eine Abgangsgruppe repräsentiert und das andere von V und W Mercapto repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines

resultierenden Salzes in die freie Verbindung oder in ein anderes Salz und/oder Umwandeln einer resultierenden freien Verbindung, die eine salzbildende Gruppe hat, in ein Salz.

5. Verfahren zur Herstellung von Verbindungen der Formel I — gezeigt im Anspruch 1 — und Salzen dieser Verbindungen, die eine salzbildende Gruppe haben, bestehend im Kondensieren einer Verbindung der Formel XIV,

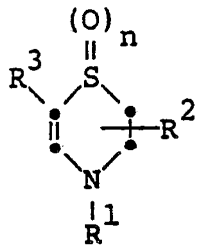

XIV

worin $R^1$, $R^2$ und n die im Anspruch 1 definierte Bedeutung haben, $R^3$ Carboxy oder funktionalisiertes Carboxy repräsentiert, mit einer Verbindung der Formel Ar-M, worin Ar die im Anspruch 1 definierte Bedeutung hat und M Alkalimetall oder Halomagnesium repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

6. Verfahren zur Herstellung von Verbindungen der Formel I — gezeigt im Anspruch 1 — und Salzen dieser Verbindungen, die eine salzbildende Gruppe haben, bestehend im Reduzieren einer Verbindung der Formel XV,

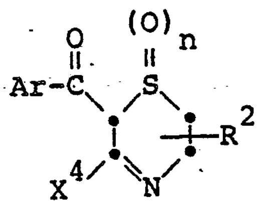

XV

worin Ar, $R^2$ und n die im Anspruch 1 definierte Bedeutung haben, $X^4$ Hydroxy oder eine Abgangsgruppe repräsentiert, unter Eliminationsbedingungen, um eine Verbindung der Formel I zu erhalten, worin $R^1$ Wasserstoff repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

7. Verfahren zur Herstellung von Verbindungen der Formel I — gezeigt im Anspruch 1 — und Salzen dieser Verbindungen, die eine salzbildende Gruppe haben, bestehend im Eliminieren von $HX^5$ aus einer Verbindung der Formel XVI,

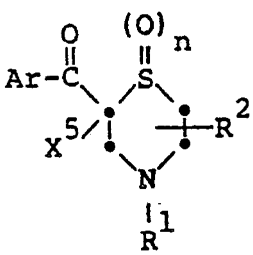

XVI

worin Ar, $R^1$, $R^2$ und n die im Anspruch 1 definierte Bedeutung haben und $X^5$ eine Abgangsgruppe repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

8. Verfahren zur Herstellung von Verbindungen der Formel I — gezeigt im Anspruch 1 — und Salzen

dieser Verbindungen, die eine salzbildende Gruppe haben, bestehend im Cyclisieren einer Verbindung der Formel XVII

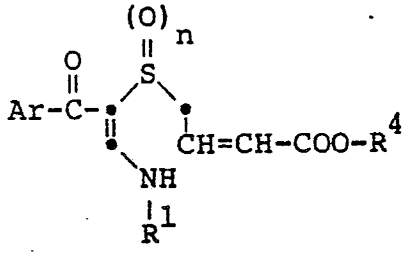

XVII

worin Ar, $R^1$ und n die im Anspruch 1 definierte Bedeutung haben und $R^4$ $C_{1-4}$-Alkyl repräsentiert; um eine Verbindung der Formel I zu erhalten, worin $R^2$ $C_{1-4}$-Alkoxycarbonylmethyl repräsentiert, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

9. Verfahren zur Herstellung von Verbindungen der Formel I — gezeigt im Anspruch 1 — und Salzen dieser Verbindungen, die eine salzbildende Gruppe haben, bestehend im Sättigen der $R^2$-substituierten Doppelbindung in einer Verbindung der Formel XVIII,

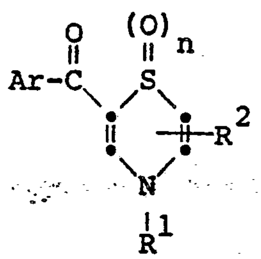

XVIII

worin Ar, $R^1$, $R^2$ und n die im Anspruch 1 definierte Bedeutung haben, und, wenn erforderlich, vorübergehenden Schützen störender reaktiver funktioneller Gruppen; und, falls gewünscht, Umwandeln einer resultierenden Verbindung in eine andere Verbindung der Formel I und/oder Umwandeln eines resultierenden Salzes in die freie Verbindung oder in ein anderes Salz und/oder Umwandeln einer resultierenden freien Verbindung, die eine salzbildende Gruppe hat, in ein Salz.

10. Verfahren nach einem der Ansprüche 1 bis 9 zur Herstellung einer Verbindung der Formel I nach Anspruch 1, worin Ar Pyridyl oder Phenyl, das substituiert ist durch Halogen, Trifluormethyl, $C_{1-4}$-Alkoxy oder Carboxy-$C_{1-4}$-alkyl ist, n gleich 0, 1 oder 2 ist, $R^1$ für Wasserstoff, $C_{1-4}$-Alkyl, Heterocyclyl-$C_{2-4}$-alkyl, Di-$C_{1-4}$-alkylamino-$C_{2-4}$-alkyl oder $C_{1-4}$-Alkanoyl steht und $R^2$ Wasserstoff oder Carboxy-$C_{1-4}$-alkyl ist, und eines pharmazeutisch annehmbaren Salzes einer solchen Verbindung, die eine salzbildende Gruppe hat.

11. Verfahren nach einem der Ansprüche 1 bis 9 zur Herstellung einer Verbindung der Formel I nach Anspruch 1, worin Ar 3-Pyridyl repräsentiert, n gleich 0 ist, $R^1$ für Wasserstoff, $C_{1-4}$-Alkyl oder $C_{1-4}$-Alkanoyl steht, $R^2$ Wasserstoff ist, oder eine Verbindung der Formel I, worin Ar Phenyl ist, das durch Halogen monosubstituiert ist, oder disubstituiertes Phenyl, wobei ein Substituent Trifluormethyl oder Halogen ist, der andere Halogen, worin n gleich 0, 1 oder 2 ist, $R^1$ Wasserstoff, $C_{1-4}$-Alkyl oder $C_{1-4}$-Alkanoyl repräsentiert und $R^2$ Wasserstoff ist, und eines pharmazeutisch annehmbaren Salzes einer solchen Verbindung, die eine salzbildende Gruppe hat.

12. Verfahren nach einem der Ansprüche 1 bis 9 zur Herstellung einer Verbindung der Formel I nach Anspruch 1, worin Ar durch Fluor oder Chlor monosubstituiertes Phenyl ist oder disubstituiertes Phenyl, wobei ein Substituent Trifluormethyl oder Fluor oder Chlor ist, der andere Fluor oder Chlor, n null ist und $R^1$ und $R^2$ Wasserstoff sind.

13. Verfahren nach einem der Ansprüche 1 bis 9 zur Herstellung einer Verbindung der Formel I nach Anspruch 1, worin Ar 4-Fluorphenyl, n null und $R^1$ und $R^2$ Wasserstoff sind, die 5,6-Dihydro-1,4(4H)-thiazin-2-yl-4-fluorphenyl-keton ist.

14. Verfahren zur Herstellung einer pharmazeutischen Zusammensetzung, worin ein Produkt, wie es in einem der Ansprüche 1 oder 10 bis 13 beansprucht wird, mit einen pharmazeutischen Träger aufgearbeitet wird.

15. Verfahren zur Herstellung einer pharmazeutischen Zusammensetzung, worin ein Produkt, wie es im Anspruch 1 beansprucht wird, worin Ar Phenyl, n gleich 0, 1 oder 2 und $R^1$ und $R^2$ Wasserstoff sind, mit einem pharmazeutischen Träger aufgearbeitet wird.

16. Verfahren zur Herstellung einer Verbindung der Formel II

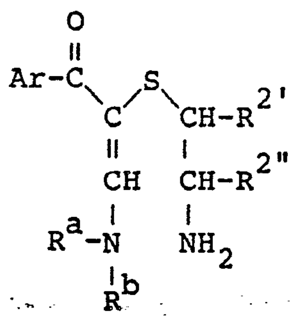

II

worin Ar Pyridyl ist oder Phenyl, das substituiert ist durch Halogen, Halo-$C_{1-4}$-alkyl, $C_{1-4}$-Alkoxy oder Carboxy-$C_{1-4}$-alkyl, $R^a$ und $R^b$ $C_{1-4}$-Alkyl sind, $R^{2'}$ und $R^{2''}$ Wasserstoff sind oder eines von $R^{2'}$ und $R^{2''}$ Wasserstoff ist und das andere Carboxy-$C_{1-4}$-alkyl oder $C_{1-4}$-Alkoxycarbonyl-$C_{1-4}$-alkyl, bestehend im Umsetzen einer Verbindung der Formel V,

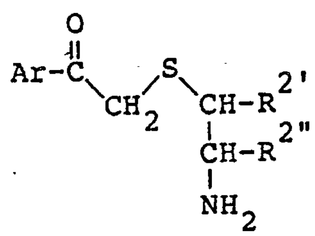

V

worin Ar, $R^{2'}$ und $R^{2''}$ wie im Anspruch 1 definiert sind und die Aminogruppe durch eine herkömmliche Amino-Schutzgruppe geschützt ist, mit einem reaktiven funktionellen Derivat von N,N-Di-$C_{1-4}$-alkylformamid.

17. Verfahren zur Herstellung einer Verbindung der Formel II — gezeigt im Anspruch 16 — bestehend im Umsetzen einer Verbindung der Formel VII,

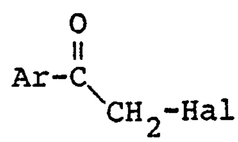

VII

worin Ar wie im Anspruch 1 definiert ist und Hal Brom oder Iod, vorzugsweise Chlor ist, mit einer Ethylenepisulfidverbindung der Formel

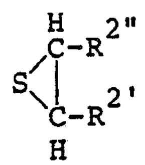

worin $R^{2'}$ und $R^{2''}$ wie im Anspruch 1 definiert sind, Umsetzen des erhaltenen Salzes der Formel

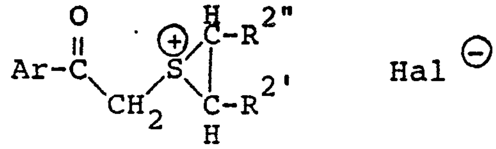

mit dem Formamid-Acetal der Formel VIb,

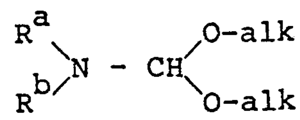

VIb

worin $R^a$ und $R^b$ niederes Alkyl sind und alk für Methyl oder Ethyl steht, um eine Verbindung der Formel

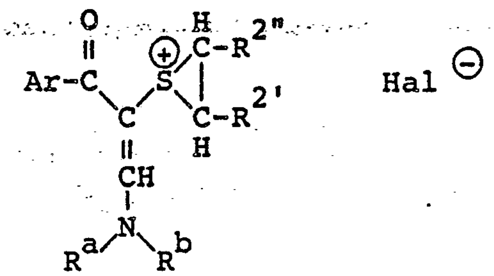

zu geben, di mit Ammoniak umgesitzt wird, um eine Verbindung der Formel II zu geben.

**Revendications pour les Etats contractants: BE CH DE FR GB IT LI LU NL SE**

1. Un composé de formule I

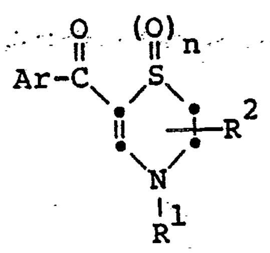

I

dans laquelle Ar représente un groupe pyridyle, phényle ou phényle substitué par des halogènes, des groupes halogénoalkyle en $C_1$—$C_4$, alcoxy en $C_1$—$C_4$ ou carboxyalkyle en $C_1$—$C_4$, n est égal à 0, 1 ou 2, $R^1$ représente l'hydrogène, un groupe alkyle en $C_1$—$C_4$, hétérocyclylalkyle en $C_2$—$C_4$, aminoalkyle en $C_2$—$C_4$, (alkyle en $C_1$—$C_4$), amino-alkyle en $C_2$—$C_4$, di-(alkyle en $C_1$—$C_4$)-amino-alkyle en $C_2$—$C_4$ ou alcanoyle en $C_1$—$C_4$ et $R^2$ représente l'hydrogène, un groupe carboxy-alkyle en $C_1$—$C_4$ ou (alcoxy en $C_1$—$C_4$)-carbonyl-alkyle en $C_1$—$C_4$, à l'exception de la 5,6-dihydro-1,4(4H)-thiazine-2-yl-phényl-cétone, du sulfoxyde et de la sulfone correspondants, et un sel d'un tel composé contenant un groupe salifiable.

2. Un composé de formule I selon la revendication 1, dans laquelle Ar représente un groupe pyridyle ou phényle substitué par des halogènes, des groupes trifluorométhyle, alcoxy en $C_1$—$C_4$ ou carboxy-alkyle en $C_1$—$C_4$, n est égal à 0, 1 ou 2, $R^1$ représente l'hydrogène, un groupe alkyle en $C_1$—$C_4$, hétérocyclyl-alkyle en $C_2$—$C_4$, di-(alkyle en $C_1$—$C_4$)-amino-alkyle en $C_2$—$C_4$ ou alcanoyle en $C_1$—$C_4$, et $R^2$ représente l'hydrogène ou un groupe carboxy-alkyle en $C_1$—$C_4$, et un sel acceptable pour l'usage pharmaceutique d'un tel composé contenant un group salifiable.

3. Un composé de formule I selon la revendication 1, dans laquelle Ar représente un groupe 3-pyridyle, n est égal à 0, $R^1$ représente l'hydrogène, un groupe alkyle en $C_1$—$C_4$ ou alcanoyle en $C_1$—$C_4$, et $R^2$ représente l'hydrogène, ou bien un composé de formule I dans laquelle Ar représente un groupe phényle monosubstitué par un halogène, ou un groupe phényle disubstitué, l'un des substituants étant un groupe trifluorométhyle ou un halogène et l'autre un halogène, dans laquelle n est égal à 0, 1 ou 2, $R^1$ représente l'hydrogène, un groupe alkyle en $C_1$—$C_4$ ou alcanoyle en $C_1$—$C_4$, et $R^2$ représente l'hydrogène, et un sel acceptable pour l'usage pharmaceutique d'un tel composé contenant un groupe salifiable.

4. Un composé de formule I selon la revendication 1, dans laquelle Ar représente un groupe phényle monosubstitué par le fluor ou le chlore, ou un groupe phényle disubstitué, l'un des substituants étant un groupe trifluorométhyle ou le fluor ou le chlore, et l'autre le fluor ou le chlore, n est égal à 0, et $R^1$ et $R^2$ représentent l'hydrogène.

5. Un composé de formule I selon la revendication 1, dans laquelle Ar représente un groupe 4-fluorophényle, n est égal à 0, et $R^1$ et $R^2$ représentent l'hydrogène, à savoir la 5,6-dihydro-1,4(4H)-thiazine-2-yl-4-fluorophényl-cétone.

6. Une composition pharmaceutique contenant en quantité antirhumatismale efficace un composé de formule I selon la revendication 1 ou un sel acceptable pour l'usage pharmaceutique d'un tel composé avec un adjuvant ou véhicule acceptable pour l'usage pharmaceutique.

7. Une composition pharmaceutique contenant une quantité antirhumatismale efficace d'un composé de formule I dans laquelle Ar représente un groupe phényle, n est égal à 0, 1 ou 2 et $R^1$ et $R^2$ représentent l'hydrogène, avec un adjuvant ou véhicule acceptable pour l'usage pharmaceutique.

8. Une composé de formule I selon la revendication 1, pour l'utilisation dans nun procédé thérapeutique pour le traitement des humains et des animaux.

9. Un composé de formule I selon la revendication 1, dans laquelle Ar représente un groupe phényle, n est égal à 0, 1 ou 2 et $R^1$ et $R^2$ représentent l'hydrogène, pour l'utilisation dans un procédé thérapeutique pour le traitement des humains et des animaux.

10. Un composé de formule I selon la revendication 1, en tant qu'agent antirhumatismal.

11. Un composé de formule I selon la revendication 1, dans laquelle Ar représente un groupe phényle, n est égal à 0, 1 ou 2 et $R^1$ et $R^2$ représentent l'hydrogène, en tant qu'agent antirhumatismal.

12. L'utilisation d'un composé de formule I selon la revendication 1, dans la préparation d'un composition pharmaceutique.

13. L'utilisation d'un composé de formule I selon la revendication 1, dans laquelle Ar représente un groupe phényle, n est égal à 0, 1 ou 2 et $R^1$ et $R^2$ représentant l'hydrogène, dans la préparation d'une composition pharmaceutique.

14. L'utilisation d'un composé de formule I selon la revendication 1, pour la préparation d'un médicamment destiné à des applications antirhumatismales.

15. L'utilisation d'un composé de formule I selon la revendication 1, dans laquelle Ar représente un groupe phényle, n est égal à 0, 1 ou 2 et $R^1$ et $R^2$ représentent l'hydrogène, pour la préparation d'un médicament destiné à des applications antirhumatismales.

16. Un composé de formule II

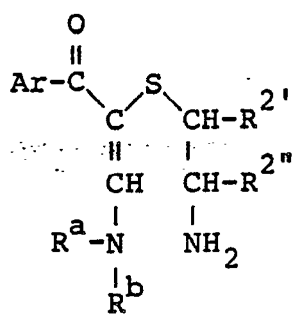

II

dans laquelle Ar représente un groupe pyridyle ou phényle substitué par des halogènes, des groupes halogénoalkyle en $C_1$—$C_4$, alcoxy en $C_1$—$C_4$ ou carboxy-alkyle en $C_1$—$C_4$, $R^a$ et $R^b$ représentant des groupes alkyle en $C_1$—$C_4$, $R^{2'}$ et $R^{2''}$ représentent l'hydrogène, ou bien l'un de ces symboles représente l'hydrogène et l'autre un groupe carboxy-alkyle en $C_1$—$C_4$ ou (alcoxy en $C_1$—$C_4$)-carbonyl-alkyle en $C_1$—$C_4$.

17. Procédé de préparation d'une composition pharmaceutique, selon lequel on combine un produit tel que revendiqué dans l'une quelconque des revendications 1 à 5 avec un véhicule pharmaceutique.

18. Procédé de préparation d'une composition pharmaceutique, selon lequel on combine un produit selon la revendication 1 dans lequel Ar représente un groupe phényle, n est égal à 0, 1 ou 2 et $R^1$ et $R^2$ représentent l'hydrogène, avec un véhicule pharmaceutique.

19. Procédé de préparation des composés de formule I selon la revendication 1 et des sels de ces composés qui contiennent un groupe salifiable, qui consiste:
à cycliser un composé de formule II

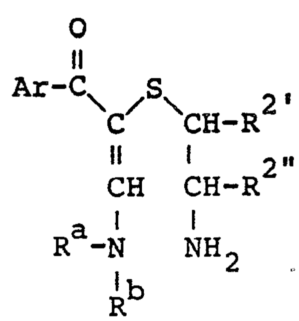

II

dans laquelle Ar a les significations indiquées dans la revendication 1, $R^a$ et $R^b$ représentent des groupes alkyle en $C_1$—$C_4$, $R^{2'}$ et $R^{2''}$ représentent l'hydrogène ou bien l'un de ces symboles représente l'hydrogène et l'autre un groupe carboxy-alkyle en $C_1$—$C_4$ ou (alcoxy en $C_1$—$C_4$)-carbonyl-alkyle en $C_1$—$C_4$, et dans

lequel le groupe amino est à l'état libre ou protégé par un groupe protecteur usuel des groupes amino, en présence d'une base, et si on le désire, on convertit le composé obtenu en un autre composé de formule I et/ou on convertit un sel ainsi obtenu en le composé libre ou en un sel différent et/ou convertit un composé libre ainsi obtenu et contenant un groupe salifiable en un sel.

20. Procédé de préparation des composés de formule I selon la revendication 1 et des sels de ces composés qui contenant un groupe salifiable, qui consiste:
à cycliser un composé de formule XI

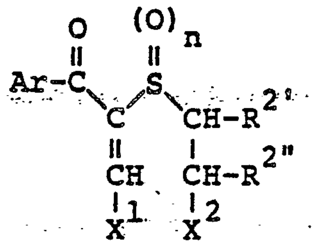

XI

dans laquelle Ar et n ont les significations indiquées dans la revendication 1, $R^{2'}$ et $R^{2''}$ représentent l'hydrogène ou bien l'un de ces symboles représente l'hydrogène et l'autre un groupe carboxy-alkyle en $C_1$—$C_4$ ou (alcoxy en $C_1$—$C_4$)-carbonyl-alkyle en $C_1$—$C_4$, l'un des symboles $X^1$ et $X^2$ représente un groupe éliminable et l'autre un groupe $NHR^1$ dans lequel $R^1$ a les significations indiquées dans la revendication 1, en protégeant transitoirement lorsque c'est nécessaire les groupes fonctionnels réactifs gênants éventuels; et si on le désire, on convertit le composé obtenu en un autre composé de formule I et/ou on convertit un sel ainsi obtenu en le composé libre ou en un sel différent et/ou on convertit un composé libre ainsi obtenu et contenant un groupe salifiable en un sel.

21. Procédé de préparation des composés de formule I selon la revendication 1 et des sels de ces composés contenant un groupe salifiable, qui consiste à condenser un composé de formule III

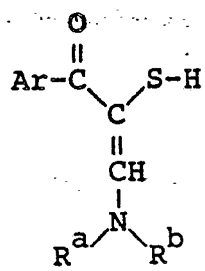

III

dans laquelle Ar et $R^a$ et $R^b$ ont les significations indiquées dans la revendication 19, et dans lequel le groupe mercapto est à l'état libre ou protégé par un groupe protecteur usuel des groupes mercapto, avec un composé de formule IV

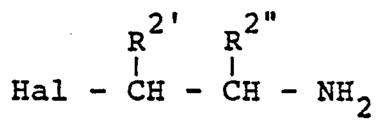

IV

dans laquelle Hal représente le chlore, le brome ou l'iode et $R^{2'}$ et $R^{2''}$ ont les significations indiquées dans la revendication 19, ou avec un sel d'un tel composé formé par addition avec un acide, en présence d'une base, et si on le désire, à convertir un composé ainsi obtenu en un autre composé de formule I et/ou à convertir un sel ainsi obtenu en le composé libre ou en un sel différent et/ou à convertir un composé libre ainsi obtenu et contenant un groupe salifiable en un sel.

22. Procédé de préparation des composés de formule I selon la revendication 1 et des sels de ces composés qui contiennent un groupe salifiable, qui consiste à condenser un composé de formule XII

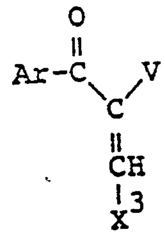

XII

avec un composé de formule XIII

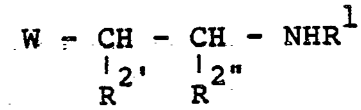

XIII

les symboles de ces formules ayant les significations suivantes: dans la formule XII, Ar a les significations indiquées dans la revendication 1 et $X^3$ représente un groupe éliminable, dans la formule XIII, $R^{2'}$ et $R^{2''}$ ont les significations indiquées dans la revendication 20 et $R^1$ les significations indiquées dans la revendication 1; et dans les formules XII et XIII, l'un des symboles V et W représente un groupe éliminable et l'autre un groupe mercapto, en protégeant transitoirement lorsque c'est nécessaire les groupes fonctionnels réactifs gênants éventuels; et si on le désire, à convertir un composé obtenu en un autre composé de formule I et/ou à convertir un sel ainsi obtenu en le composé libre ou en un sel différent et/ou à convertir un composé obtenu à l'état libre et contenant un groupe salifiable en un sel.

23. Procédé de préparation des composés de formule I selon la revendication 1 et des sels de ces composés contenant un groupe salifiable, qui consiste à condenser un composé de formule XIV

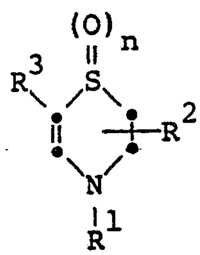

XIV

dans laquelle $R^1$, $R^2$ et n ont les significations indiquées dans la revendication 1, et $R^3$ représente un groupe carboxy ou un groupe carboxy à l'état de dérivé fonctionnel, avec un composé de formule Ar-M dans laquelle Ar a les significations indiquées dans la revendication 1 et M représente un métal alcalin ou un groupe halogénomagnésium, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

24. Procédé de préparation des composés de formule I selon la revendication 1 et des sels de ces composés qui contenant un groupe salifiable, qui consiste à réduire un composé de formule XV

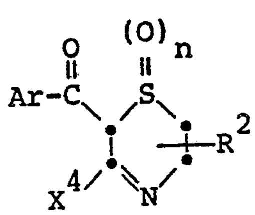

XV

dans laquelle Ar, $R^2$ et n ont les significations indiquées dans la revendication 1, $X^4$ représente un groupe hydroxy ou un groupe éliminable, dans des conditions propres à l'élimination, afin d'obtenir un composé de formule I dans laquelle $R^1$ représente l'hydrogène, en protégeant transitoirement lorsque c'est nécessaire les groupes fonctionnels réactifs éventuels gênants; et si on le désire, à convertir un composé ainsi obtenu en un autre composé de formule I et/ou à convertir un sel ainsi obtenu en le composé libre ou

en un sel différent et/ou à convertir un composé obtenu à l'état libre et contenant un groupe salifiable en un sel.

25. Procédé de préparation des composés de formule I selon la revendication 1 et des sels de ces composés qui contiennent un groupe salifiable, qui consiste à éliminer $HX^5$ d'un composé de formule XVI

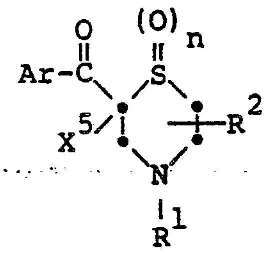

XVI

dans laquelle Ar, $R^1$, $R^2$ et n ont les significations indiquées dans la revendication 1 et $X^5$ représente un groupe éliminable, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

26. Procédé de préparation des composés de formule I selon la revendication 1 et des sels de ces composés qui contiennent un groupe salifiable, qui consiste: à cycliser un composé de formule XVII

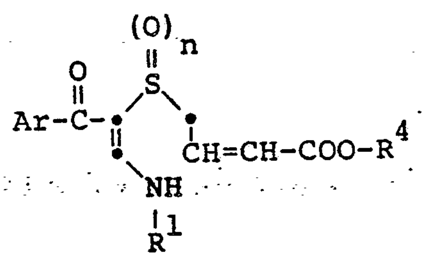

XVII

dans laquelle Ar, $R^1$ et n ont les significations indiquées dans la revendication 1 et $R^4$ représente un groupe alkyle en $C_1$—$C_4$, afin d'obtenir un composé de formule I dans laquelle $R^2$ représente un groupe (alcoxy en $C_1$—$C_4$)-carbonylméthyle, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

27. Procédé de préparation des composés de formule I selon la revendication 1 et des sels de ces composés qui contiennent un groupe salifiable, qui consiste à saturer la double liaison portant le substituant $R^2$ d'un composé de formule XVIII

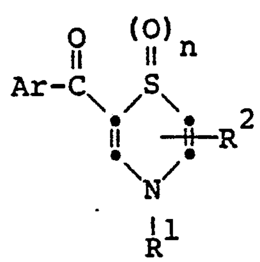

XVIII

dans laquelle Ar, $R^1$, $R^2$ et n ont les significations indiquées dans la revendication 1, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

**Revendications pour l'Etat contractant: AT**

1. Un procédé de préparation d'un composé de formule I

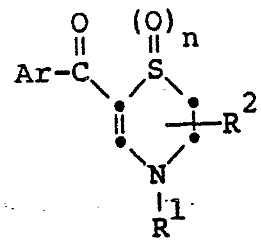

I

dans laquelle Ar représente un groupe pyridyle, phényle ou phényle substitué par des halogènes, des groupes halogénoalkyle en $C_1$—$C_4$, alcoxy en $C_1$—$C_4$ ou carboxyalkyle en $C_1$—$C_4$, n est égal à 0, 1 ou 2, $R^1$ représente l'hydrogène, un groupe alkyle en $C_1$—$C_4$, hétérocyclyl-alkyle en $C_2$—$C_4$, aminoalkyle en $C_2$—$C_4$, (alkyle en $C_1$—$C_4$), amino-alkyle en $C_2$—$C_4$, di-(alkyle en $C_1$—$C_4$)-amino-alkyle en $C_2$—$C_4$ ou alcanoyle en $C_1$—$C_4$ et $R^2$ représente l'hydrogène, un groupe carboxy-alkyle en $C_1$—$C_4$ ou (alcoxy en $C_1$—$C_4$)-carbonyl-alkyle en $C_1$—$C_4$, à l'exception de la 5,6-dihydro-1,4(4H)-thiazine-2-yl-phényl-cétone et du sulfoxyde et de la sulfone correspondants, et d'un sel d'un tel composé contenant un groupe salifiable qui consiste à cycliser un composé de formule II

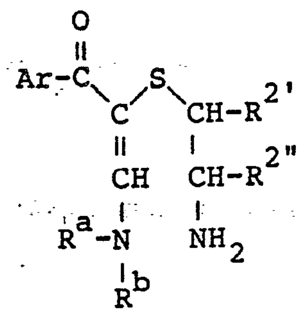

II

dans laquelle Ar a les significations indiquées ci-dessus, $R^a$ et $R^b$ représentent des groupes alkyle en $C_1$—$C_4$, $R^{2'}$ et $R^{2''}$ représentent l'hydrogène ou bien l'un de ces symboles représente l'hydrogène et l'autre un groupe carboxy-alkyle en $C_1$—$C_4$ ou (alcoxy en $C_1$—$C_4$)-carbonyl-alkyle en $C_1$—$C_4$, et dans lequel le groupe amino est à l'état libre ou protégé par un groupe protecteur usuel des groupes amino, en présence d'une base, et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

2. Un procédé de préparation des composés de formule I selon la revendication 1 et des sels de ces composés qui contiennenent un groupe salifiable, qui consiste à condenser un composé de formule III

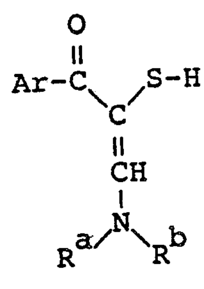

III

dans laquelle Ar, et $R^a$ et $R^b$ ont les significations indiquées dans la revendication 1, et dans lequel le groupe

mercapto es à l'état libre ou protégé par un groupe protecteur usuel des groupes mercapto, avec un composé de formule IV

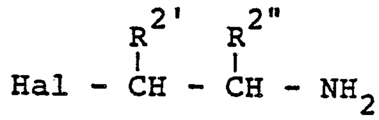

IV

dans laquelle Hal représente le chlore, le brome ou l'iode et $R^{2'}$ et $R^{2''}$ ont les significations indiquées dans la revendication 1, ou avec un sel d'un tel composé formé par addition avec un acide, en présence d'une base, et si à le désire, à convertir un composé ainsi obtenu en un autre composé de formule I et/ou à convertir un sel ainsi obtenu en le composé libre ou en un sel différent et/ou à convertir un composé obtenu à l'etat libre et contenant un groupe salifiable en un sel.

3. Un procédé de préparation des composés de formule I selon la revendication 1 et des sels de ces composés qui contiennent un groupe salifiable, qui consiste à cycliser un composé de formule XI

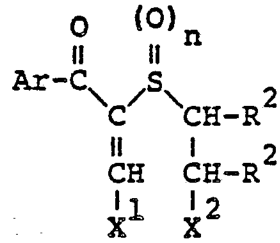

XI

dans laquelle Ar et n ont les significations indiquées dans la revendication 1, $R^{2'}$ et $R^{2''}$ représentent l'hydrogène ou bien l'un de ces symboles représente l'hydrogène et l'autre un groupe carboxy-alkyle en $C_1$—$C_4$ ou (alcoxy en $C_1$—$C_4$)-carbonyl-alkyle en $C_1$—$C_4$, l'un des symboles $X^1$ et $X^2$ représente un groupe éliminable et l'autre un groupe $NHR^1$ dans lequel $R^1$ a les significations indiquées ci-dessus, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état et contenant un groupe salifiable en un sel.

4. Un procédé de préparation des composés de formule I selon la revendication 1 et des sels de ces composés qui contiennent un groupe salifiable, qui consiste à condenser un composé de formule XII

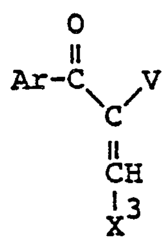

XII

avec un composé de formule XIII

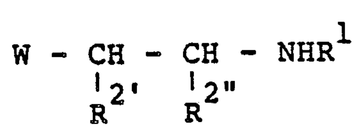

XIII

les symboles des formules XII et XIII ayant les significations suivantes dans la formule XII, Ar a les significations indiquées dans la revendication 1 et $X^3$ représente un groupe éliminable, dans la formule XIII, $R^1$, $R^{2'}$ et $R^{2''}$ ont les significations indiquées dans la revendication 1, et dans les formules XII et XIII, l'un des symboles V et W représente un groupe éliminable et l'autre un groupe mercapto, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

5. Un procédé de préparation des composés de formule I selon la revendication 1 et des sels de ces composés qui contiennent un groupe salifiable, qui consiste à condenser un composé de formule XIV

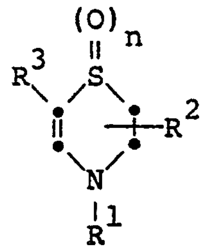

XIV

dans laquelle $R^1$, $R^2$ et n ont les significations indiquées dans la revendication 1 et $R^3$ représente un groupe carboxy ou carboxy à l'état de dérivé fonctionnel, avec un composé de formule Ar-M dans laquelle Ar a les significations indiquées dans la revendication 1 et M représente un métal alcalin ou un groupe halogénomagnésium, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

6. Un procédé de préparation des composés de formule I selon la revendication 1 et des sels de ces composés qui contiennent un groupe salifiable, qui consiste à réduire un composé de formule XV

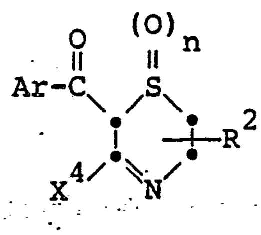

XV

dans laquelle Ar, $R^2$ et n ont les significations indiquées dans la revendication 1, $X^4$ représente un groupe hydroxy ou un groupe éliminable, dans des conditions propres à l'élimination, afin d'obtenir un composé de formule I dans laquelle $R^1$ représente l'hydrogène, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

7. Un procédé de préparation des composés de formule I selon la revendication 1 et des sels de ces composés qui contiennent un groupe salifiable, qui consiste à éliminer $HX^5$ d'un composé de formule XVI

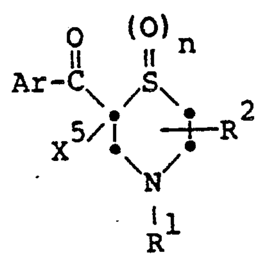

XVI

dans laquelle Ar, $R^1$, $R^2$ et n ont les significations indiquées dans la revendication 1 et $X^5$ représente un groupe éliminable, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

8. Un procédé de préparation des composés de formule I selon la revendication 1 et des sels de ces composés qui contiennent un groupe salifiable, qui consiste à cycliser un composé de formule XVII

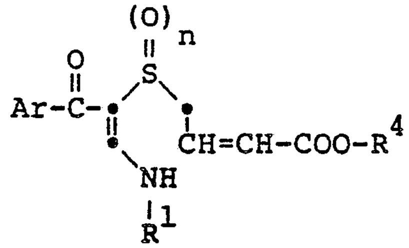

XVII

dans laquelle Ar, $R^1$ et n ont les significations indiquées dans la revendication 1 et $R^4$ représente un groupe alkyle $C_1$—$C_4$, afin d'obtenir un composé de formule I dans laquelle $R^2$ représente un groupe (alcoxy en $C_1$—$C_4$)-carbonylméthyle, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

9. Un procédé de préparation des composés de formule I selon la revendication 1 et des sels de ces composés qui contiennent un groupe salifiable, qui consiste à saturer la double liaison portant le substituant $R^2$ dans un composé de formule XVIII

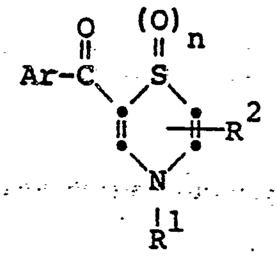

XVIII

dans laquelle Ar, $R^1$, $R^2$ et n ont les significations indiquées dans la revendication 1, en protégeant transitoirement lorsque c'est nécessaire les groupes fonctionnels réactifs gênants éventuels; et si on le désire, à convertir un composé ainsi obtenu en un autre composé de formule I et/ou à convertir un sel ainsi obtenu en le composé libre ou en un sel différent et/ou à convertir un composé obtenu à l'état libre et contenant un groupe salifiable en un sel.

10. Un procédé selon l'une quelconque des revendications 1 à 9, pour la préparation d'un composé de formula I selon la revendication 1, dans lequel Ar représente un groupe pyridyle ou phényle substitué par des halogènes, des groupes trifluorométhyle, alcoxy en $C_1$—$C_4$ ou carboxy-alkyle en $C_1$—$C_4$, n est égal à 0, 1 ou 2, $R^1$ représente l'hydrogène, un groupe alkyle en $C_1$—$C_4$, hétérocyclyl-alkyle en $C_2$—$C_4$, di-(alkyle en $C_1$—$C_4$)-amino-alkyle en $C_2$—$C_4$ ou alcanoyle en $C_1$—$C_4$, et $R^2$ représente l'hydrogène ou un groupe carboxy-alkyle en $C_1$—$C_4$, et d'un sel acceptable pour l'usage pharmaceutique d'un tel composé contenant un group salifiable.

11. Un procédé selon l'une quelconque des revendications 1 à 9, pour la préparation d'un composé de formule I selon la revendication 1, dans laquelle Ar représente un groupe 3-pyridyle, n est égal à 0, $R^1$ représente l'hydrogène, un groupe alkyle en $C_1$—$C_4$ ou alcanoyle en $C_1$—$C_4$ et $R^2$ représente l'hydrogène, ou d'un composé de formule I dans lequel Ar représente un groupe phényle monosubstitué par un halogène, ou phényle disubstitué, l'un les substituants étant un groupe trifluorométhyle ou un halogène et l'autre un halogène, n est égal à 0, 1 ou 2, $R^1$ représente l'hydrogène, un groupe alkyle en $C_1$—$C_4$ ou alcanoyle en $C_1$—$C_4$ et $R^2$ représente l'hydrogène, et d'un sel acceptable pour l'usage pharmaceutique d'un tel composé qui contient un groupe salifiable.

12. Un procédé selon l'une quelconque des revendications 1 à 9, pour la préparation d'un composé de formule I selon la revendication 1 dans lequel Ar représente un groupe phényle monosubstitué par le fluor ou le chlore ou phényle disubstitué, l'un des substituants étant un groupe trifluorométhyle ou le fluor ou le chlore et l'autre le fluor ou le chlore, n est égal à 0 et $R^1$ et $R^2$ représentent l'hydrogène.

13. Un procédé selon l'une quelconque des revendications 1 à 9, pour la préparation d'un composé de formule I selon la revendication 1 dans lequel Ar représente un groupe 4-fluorophényle, n est égal à 0 et $R^1$ et $R^2$ représentent l'hydrogène, à savoir la 5,6-dihydro-1,4(4H)-thiazine-2-yl-4-fluorophényl-cétone.

14. Un procédé de préparation d'une composition pharmaceutique, selon lequel on combine un produit tel que revendiqué dans l'une quelconque des revendications 1 ou 10 à 13 avec un véhicule pharmaceutique.

15. Procédé de préparation d'une composition pharmaceutique, selon lequel on combine un produit tel

que revendiqué dans la revendication 1 et dans lequel Ar représente un groupe phényle, n est égal à 0, 1 ou 2 et $R^1$ et $R^2$ représentent l'hydrogène, avec un véhicule pharmaceutique.

16. Un procédé de préparation d'un composé de formule II

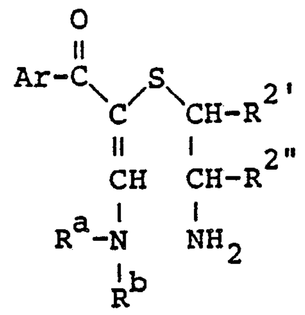

II

dans laquelle Ar représente un groupe pyridyle ou phényle substitué par des halogènes, des groupes halogénoalkyle en $C_1$—$C_4$, alcoxy en $C_1$—$C_4$ ou carboxy-alkyle en $C_1$—$C_4$, $R^a$ et $R^b$ représentent des groupes alkyle en $C_1$—$C_4$, $R^{2'}$ et $R^{2''}$ représentent l'hydrogène ou bien l'un de ces symboles représente l'hydrogène et l'autre un groupe carboxy-alkyle en $C_1$—$C_4$ ou (alcoxy en $C_1$—$C_4$)-carbonyl-alkyle en $C_1$—$C_4$, qui consisté à faire réagir un composé de formule V

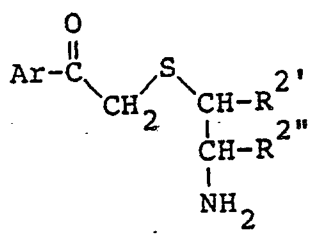

V

dans laquelle Ar, $R^{2'}$ et $R^{2''}$ ont les significations indiquées dans la revendication 1, et dans lequel le groupe amino est protégé par un groupe protecteur usuel des groupes amino, avec un dérivé fonctionnel réactif d'un N,N-di-(alkyle en $C_1$—$C_4$)-formamide.

17. Un procédé de préparation d'un composé de formule II selon la revendication 16, qui consiste à faire réagir un composé de formule VII

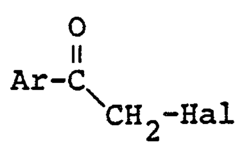

VII

dans laquelle Ar a les significations indiquées dans la revendication 1 et Hal représente le brome ou l'iode, mais de préférence le chlore, avec un épisulfure d'éthylène de formule

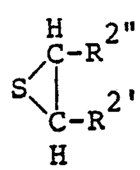

dans laquelle $R^{2'}$ et $R^{2''}$ ont les significations indiquées dans la revendication 1, ce qui donne un sel de formule

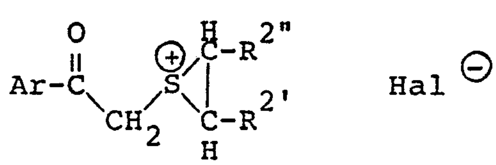

qu'on fait réagir avec l'acétal du formamide de formule VIb

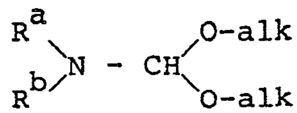

VIb

dans laquelle $R^a$ et $R^b$ représentent des groupes alkyle inférieurs et "alk" représente un groupe méthyle ou éthyl, ce qui donne un composé de formule

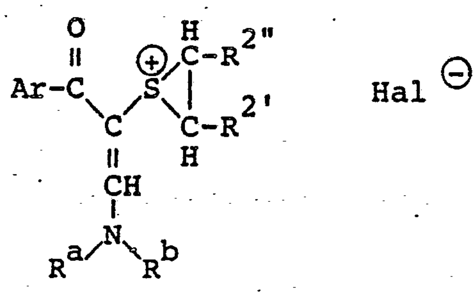

qu'on fait réagir avec l'ammoniac, ce qui donne un composé de formule II.